# EUROPEAN PATENT SPECIFICATION

(11) **EP 0 826 003 B1**
(45) Date of publication and mention of the grant of the patent: **05.09.2001**
(21) Application number: 96920118.5
(22) Date of filing: 01.05.1996
(51) Int. Cl.: C07K 14/47, G01N 33/569, A61K 38/17, A61K 39/00

(54) **COMPOSITIONS AND METHODS FOR DETECTING AND TREATING ACQUIRED IMMUNODEFICIENCY SYNDROME**
ZUSAMMENSETZUNGEN UND VERFAHREN ZUR DETEKTION UND BEHANDLUNG DES ERWORBENEN IMMUN-DEFIZIENZ-SYNDROMS
COMPOSITIONS ET PROCEDES POUR DECELER ET TRAITER LE SYNDROME IMMUNODEFFICITAIRE ACQUIS

(30) Priority: 01.05.1995 US 431883; 07.06.1995 US 485548; 01.05.1996 US 641936
(43) Date of publication of application: 04.03.1998
(60) Divisional application: 00125954.8
(73) Proprietor: Tomson, U.S.A., Ltd., South Pasadena, CA 91030 (US)
(72) Inventor: ZHABILOV, Harry, P., 1303 Sofia (BG)
(74) Representative: Rambelli, Paolo
(86) International application number: US9606079
(87) International publication number: WO9634886

(56) References cited:
- EP-A- 0 149 468
- EP-A- 0 392 315
- WO-A-89/05455
- WO-A-93/21943
- US-A- 4 415 553
- ARCHIVES OF BIOCHEMISTRY AND BIOPHYSICS, vol. 190, no. 2, 1978, pages 398-404, XP000601954 B.DAVID STOLLAR ET AL: "Separation of anti-histone antibodies from nonimmune histone precipitating serum proteines predominantly alpha-2-Macroglobulin"
- GERALD D. FASMAN PH.D.: "CRC Handbook of Biochemistry and Molecular Biology" 1989 , CRC PRESS INC. , BOCA RATON FLORIDA XP002013034 see page 294 - page 300

## Description

### TECHNICAL FIELD OF THE INVENTION

This invention relates to the area of immunology and virology, and specifically relates to compositions obtainable from mammalian thymus cells which are useful as diagnostics, vaccines, and therapeutics for human immunodeficiency virus (HIV) infection and related diseases such as acquired immunodeficiency syndrome (AIDS) and AIDS-related complex (ARC) . The diagnostic, vaccination, and therapeutic use of these compositions, and devices using these compositions are also disclosed.

### BACKGROUND OF THE INVENTION

Bone marrow produces cells which are destined to become immune cells. These cells become lymphocytes or phagocytes. Lymphocytes are small white blood cells that bear the major responsibility for carrying out the activities of the immune system. The two major classes of lymphocytes are B cells and T cells. B cells mature in the bone (thus the term "B cells") marrow. T cells migrate to the thymus (thus the term "T cells") where they multiply and mature into cells capable of immune response. Upon exiting the bone marrow and thymus, both B and T cells travel widely and continuously throughout the body.

There are two types of T cells, regulatory and cytotoxic T cells, which contribute to the immune defenses in two major ways. Chief among the T cells are "helper/inducer" cells. Identifiable by the T4 cell marker, helper T cells are essential for activating B cells and other T cells as well as natural killer cells and macrophages. Cytotoxic T cells are killer cells which, for example, directly attack and rid the body of cells that have been infected by viruses or transformed by cancer.

Important phagocytes are monocytes and macrophages. Monocytes circulate in the blood, then migrate into tissues where they develop into macrophages ("big eaters"). Macrophages are found throughout the body tissues and are versatile cells that play many roles. As scavengers, they rid the body of worn-out cells and other debris. Foremost among cells that present antigen to T cells, having first digested and processed it, macrophages play a crucial role in initiating the immune response. As secretory cells, monocytes and macrophages are vital to the regulation of immune responses. They also carry receptors for lymphokines that allow them to be "activated" to pursue microbes and tumor cells.

Acquired Immunodeficiency Syndrome (AIDS) is caused by a virus, the human immunodeficiency virus (HIV). HIV destroys helper T cells and is harbored in macrophages and monocytes. AIDS is characterized by various unusual infections and otherwise rare cancers. HIV also damages tissue of the brain and spinal cord, producing progressive dementia.

When HIV infects a human patient, it incorporates itself into the deoxyribonucleic acid (DNA) of the immune cells and for a variable period of between 3 months to 7 years, the patient may not exhibit any immunodeficiency symptoms and sometimes do not produce detectable level of antibodies against AIDS. Since an initial HIV infection may not immediately lead to detectable clinical disease symptoms or detectable level of antibodies, the term "HIV infection" as used herein encompasses both the infection and any disease resulting therefrom, the latter being termed "HIV-related diseases". Examples of HIV-related diseases are AIDS and ARC. After the above incubation period, the HIV multiplies within the infected cell and eventually bursts the host cells which release the newly formed viruses. Since the host cells are destroyed in the process, the patient's immune system is impaired and the host is susceptible to opportunistic diseases that a human with intact immune system is not susceptible to. In human, generally the AIDS virus will multiply and the human will eventually die from severe immunodeficiency. Interest-ingly, only humans suffer from AIDS. When a non-human mammal, such as a rabbit, mouse, rat or cow, is injected with HIV, the animal may temporarily have some T cells destroyed. However, 14 to 21 days post-infection, the animal would mount an antibody attack and does not succumb to AIDS. Thus, there is no animal model for AIDS.

Currently, there is no cure nor effective therapeutic for AIDS. Research in this area is underway.

There is also a continuous search for a method for accurately diagnosing AIDS at an earlier stage of the disease. Currently, the commercially available diagnostic tests are generally directed to detecting the patients' antibodies against HIV. Such tests have a window of about 14 to 21 days from the time the patient is infected with AIDS to the time the patient produces antibodies against the virus. Therefore, if a patient is tested during this window of time, the tests will produce a false negative result. On the other hand, some of these tests may also give false positive results due to non-specific binding of the antibodies. Another means for detecting the viral infection is through nucleic acid hybridization.

Unless otherwise noted, the following is based on Stein, D.S., ***et al., J. Infect. Diseases,*** **165**:352 (1992). The surrogate marker that most closely correlates with the stage of HIV infection is the CD4⁺, or T helper, cell count. HIV-1 envelope glycoprotein, gp120, specifically binds to the CD4 receptor that is expressed in greatest concentration in a subset of T lymphocytes and in lower amounts on monocytes and macrophages. Cells expressing CD4 receptors are termed the "helper/inducer" subset, reflecting their role as both helper cells for B cell responses for antigens expressed on cells bearing human leukocyte antigen (HLA) class II receptors and inducer cells that cause T cells to suppress immune responses. The selective loss of CD4⁺ cells results in numerous immune defects associated with susceptibility to the opportunistic infections that are the hallmark of AIDS.

The HIV core antigen p24 can be detected before the appearance of HIV antibodies. After the appearance of HIV antibodies by the screening enzyme-linked immunosorbent assay (ELISA), p24 antigenemia generally becomes undetectable, though it can occasionally persist and often will recur later in the disease. HIV-I titers found in plasma and peripheral blood mononuclear cell cultures also fall rapidly as specific antibodies are detectable, suggesting at least a transiently effective host immune response. Markers of immune stimulation includes β₂-microglobulin.

In patients followed from the time of seroconversion, CD4⁺ cell decline has been correlated with progression to AIDS. Serum levels of β₂-microglobulin and detection of p24 antigen in blood were also both independently correlated with rates of progression. Combined with CD4⁺ cell counts, use of β₂-microglobulin and p24 antigen increased prognostic accuracy for progression to AIDS compared with CD4⁺ cell count alone.

However, it was rare for seroconverters to have a consistent decline in their percentage of CD4⁺ cells over the next three years. In the interval between visits, stable or declining levels of CD4⁺ cell percentages were found in 38% of subjects, with 12% experiencing declines followed by a leveling in their rates of loss of CD4⁺ cells. Overall, 62% experienced declines in their CD4⁺ cell percentage over three years of follow-up.

In a study of 306 HIV-infected seropositive homosexual men with unknown times of seroconversion, both a CD4⁺ cell count < 500/*µ*l and p24 antigen detection were predictive of AIDS within 30 months.

Increased CD8⁺ cell counts were found to be somewhat predictive of subsequent development of AIDS.

To better correlate clinical end points, such as survival and progression to AIDS, with surrogate markers of antiviral therapy effects, analysis of additional markers such as neopterin and β₂-microglobulin, among others, have been combined with the CD4⁺ cell count and p24 antigen.

In a limited study {Jacobson, M.A., *BNJ,* **302**:73 (1991) } of patients with AIDS and ARC who tolerated an anti-AIDS drug, zidovudine, and who survived for 12 weeks, the following was found.

After controlling for three factors (age, diagnosis of AIDS at baseline, log of the baseline serum neopterin concentration), the log of the CD4⁺ cell count at 8-12 weeks, but not the change over time, best predicted subsequent survival. A decrease in β₂-microglobulin concentration at 8-12 weeks significantly predicted survival and, combined with the log of the CD4⁺ cell count, provided the best predictive model. Decreases in p24 antigenemia, serum neopterin concentrations, and the Karnofsky performance status (a measure of function in routine activities) did not significantly correlate with survival on therapy.

Stein, D. S., ***et al.***, above, conclude that changes in CD4⁺ cell counts and other surrogate markers may be increasingly used as the sole end point for investigations of antiretroval activity, of a drug or therapy, in patients with early HIV infection.

### SUMMARY OF THE INVENTION

One aspect of the invention presents compositions useful for diagnosing and treating HIV infections such as AIDS and ARC.

Another aspect of the invention presents methods for detecting HIV infection using the above compositions.

Another aspect of the invention presents methods for treating HIV infection using the above compositions.

Another aspect of the invention presents methods for preparing the above composition from thymus cells of non-human mammals.

Another aspect of the invention presents devices for ***in vitro*** detection of HIV infection using the above compositions.

### BRIEF DESCRIPTION OF THE DRAWINGS

FIG. 1 presents the chromatogram of the Thymus Factor ("TF") preparation.

FIG. 2 graphically presents the placement of the two-dimensional gel in its first-dimension.

FIG. 3 graphically presents the placement of the two-dimensional gel in its second-dimension.

FIGs. 4 to 11, are photographs of two-dimensional gels showing the precipitation patterns of TF with serum samples from HIV-positive or AIDS human subjects and a healthy human subject.

FIG. 12 graphically presents FIG. 7, and the distances measured for its analysis.

FIG. 13 is a photograph of a two-dimensional electrophoretic gel showing the precipitation pattern of TF with a serum sample from an HIV-negative human subject.

FIG. 14 is a photograph of a two-dimensional electrophoretic gel showing the precipitation pattern of TF with a serum sample from an HIV-positive human subject.

FIG. 15 is a photograph of a two-dimensional electrophoretic gel of the serum sample collected on May 4, 1995 from the test subject of Example 3.

FIG. 16 is a photograph of a two-dimensional electrophoretic gel of the serum sample collected on May 11, 1995 from the test subject of Example 3.

FIG. 17 is a photograph of a two-dimensional electrophoretic gel of the serum sample collected on May 18, 1995 from the test subject of Example 3.

FIG. 18 is a photograph of a two-dimensional electrophoretic gel of the serum sample collected on May 24, 1995 from the test subject of Example 3.

FIG. 19 is a photograph of a two-dimensional electrophoretic gel of the serum sample collected on May 31, 1995 from the test subject of Example 3.

### DESCRIPTION OF THE INVENTION

The present invention presents a protein, herein referred to as Thymus Factor ("TF"), which is useful for the detection of HIV which causes the fragmentation of another protein, herein referred to as aTF, which is capable of being bound to TF.

It is postulated that HIV causes the fragmentation of aTF. Thus, an individual uninfected by the HIV will have intact aTF, whereas an individual infected with HIV will have fragments of aTF. It is further postulated that, at an earlier stage of infection, the amount of aTF that are fragmented are less than at a later stage of infection. Similarly, the fragments are smaller in size in later stages of infection. Thus, detection of aTF fragments indicates an infection by HIV. The amount and size of the aTF fragments indicate the stage of the infection. aTF and its fragments can be detected by their precipitation patterns with TF, in particular, by the locations of the precipitates in relation to β-, α₁- and α₂- macroglobulins, ***e.g.***, in a gel electrophoresis, as described in EXAMPLE 2 below.

It was observed on a two-dimensional electrophoretic gel that TF interacted with serum from a human uninfected with HIV to form a continuous precipitation spur with β-, α-, and **α**₂ macroglobulins in the serum. The precipitation spur was broken or absent alongside one or more of the macroglobulins when TF interacted with serum from HIV-infected humans. It is postulated that TF precipitates aTF, found in the β-, α₁-, and α₂- macroglobulins of healthy humans, which links the β-, α₁-, and α₂-macroglobulins and thus the precipitation spur is continuous alongside β-, α₁-, and α₂- macroglobulins. In the case of HIV-infected humans, aTF is fragmented, thus causing broken precipitation spurs or the absence of precipitation spurs with the macroglobulins.

Thus, TF precipitates with aTF found in human biological samples. Examples of the biological samples are bodily fluids such as: blood, serum and plasma. Besides the two-dimensional gel electrophoresis described in EXAMPLE 2, the precipitation pattern can be detected using a number of techniques known to one skilled in the art, including methods used for detecting the immunoprecipitation pattern between antibodies and their antigen, examples of such techniques are described in Oudin, J., ***C.R. Acad Sci.***, **222**:115 (1946); Oudin, J., ***Methods in Medical Research*****, V**:335-78, Corcoran A.C., ed., Year Book Publishers Inc. (1952); Ouchterlony, O., ***Acta. Path. Microbiol. Scand.***, **25**:186 (1948); Ouchterlony, O., ***Progress in Allergy,*** **V**:1-78, Kallos P. & Wakeman B.H., Basel and Karger, New York (1958); Ouchterlony, O., ***Progress in Allergy,*** **VI**:30-154, Kallos P. & Wakeman B.H., ed., Basel and Karger, New York (1962) ; Mancini, G. *et al., Prot. Biol. Fluids 11th Colloqu. Bruges*, pp. 370-3, Peters, H., ed. (1964) ; Mancini, G. *et al., Immunochemistry,* **2**:235 (1965).

TF can also be used to treat HIV infections, such as AIDS and ARC. TF can exist in a methylated or an unmethylated state. The term "TF" as used herein, unless otherwise modified, *e.g.* as "methylated TF", covers both the methylated and unmethylated TF.

The present invention also presents methods for obtaining TF, in particular, from thymus cells. The thymus cells are preferably from non-human mammals regardless of whether they are infected by HIV, though HIV infected non-human mammals have higher titers of TF. Examples of the non-human mammals are: monkeys, gorillas, chimpanzees, guinea pigs, cows, rabbits, dogs, mice and rats.

Both methylated and unmethylated TF can bind aTF and thus can be used to diagnose HIV infection, in particular AIDS. However, at least in the case of sera from human test subjects, the methylated TF suffers less from non-specific binding with other proteins, compared to the unmethylated TF. Thus, methylated TF is preferred. Unmethylated TF can be chemically methylated using methods known in the art, for example, as described in EXAMPLE 1 below. Methylated TF can be used as a therapeutic for HIV infection, in particular AIDS.

TF is preferably purified from the thymus cells of freshly sacrificed, *i.e.*, 4 hours or less after sacrifice, mammals such as monkeys, gorillas, chimpanzees, guinea pigs, cows, rabbits, dogs, mice and rats. Histones of thymic origin are known from the prior art as being able to precipitate serum proteins, e.g. α₂-macroglobulin (Arch. Biochem. Biophys.190(2):398-404(1978)) as well as exhibiting hormone like activities (EP-A-392315 and EP-A-149468) and antineoplastic properties (CRC Handbook of Biochemistry and Molecular Biology (3rd ed.), Gerald D. Frasman (ed.), Boca Raton (FL), 1989, Proteins (Volume II) pages 294-300).

The nuclei from the thymus cells are isolated using method known in the art. Part of their lysine-rich histone fractions are extracted using the pepsin degradation method of US Patent No. 4,415,553. Other degradative methods such as trypsin degradation, BrCN degradation appear ineffective in extracting TF. The protein rich fragment of the isolate is purified by cation exchange chromatography. The proteins in the TF fractions (methylated and unmethylated) can be concentrated by any one of several techniques well known to those skilled in the art, including high salt precipitation, for example, with ammonium sulfate, or by ultrafiltration. If TF is concentrated by precipitation, it is preferably subsequently resuspended in a suitable physiologically balanced salt solution containing protease inhibitor(s).

### TF Administration As Therapeutics Against HIV Infections

Applicants observed that non-human mammals, such as cows, which were infected with HIV but did not develop AIDS, have intact TF. This invention postulates that TF plays a role in preventing the progression of HIV infections, in particular AIDS. Therefore, it would be therapeutic to administer TF to HIV infected human. The TF is preferably from non-human mammals which do not develop AIDS when infected with HIV. Methylated non-mammalian TF is preferred. Further, it is preferable to provoke an immunoresponse in the infected human under treatment. To that end, TF is preferably delivered with an adjuvant, or TF is chemically conjugated to an immunogenic carrier that would provoke an antibody response in the patient. Examples of immunogenic carriers are: keyhole limpet and bovine serum albumin.

In one aspect of the invention, TF is used to vaccinate against, to treat, or cure HIV infections, especially AIDS and ARC. For example, TF is prepared for administration by mixing it at the desired degree of purity with adjuvants or physiologically acceptable carriers, *i.e.* carriers which are nontoxic to recipients at the dosages and concentrations employed. Although TF is desirably administered with an adjuvant, in situations where the initial inoculation is delivered with TF, boosters with TF may not require adjuvant.

Injections (intramuscular or subcutaneous) will be the primary route for therapeutic administration of the vaccines of this invention. However, intravenous delivery, delivery through catheter, or other surgical tubing may also be used. Alternative routes include tablets and the like, liquid formulations, and inhalation of lyophilized or aerosolized receptors. Liquid formulations may be utilized after reconstitution from powder formulations.

TF may also be administered via microspheres, liposomes, other microparticulate delivery systems or sustained release formulations placed in certain tissues including blood.

The dosage of TF administered will depend upon the properties of the formulation employed, ***e.g.*** its binding activity and ***in vivo*** plasma half-life, the concentration of TF in the formulation, the administration route, the site and rate of dosage, the clinical tolerance of the patient involved, the pathological condition afflicting the patient and the like, as is well within the skill of the physician. Different dosages are utilized during a series of sequential inoculations; the practitioner may administer an initial inoculation and then boost with relatively smaller doses of TF.

The following is an example of a TF formulation, dosage and administration schedule. The patient is administered an intramuscular or subcutaneous injection containing 8 mg of TF (preferably 2 ml of a formulation containing 4 mg/ml of TF in a physiologically acceptable solution) or 57 *µ*g of TF protein per 1 kg body weight of the patient. Each treatment course consists of 16 injections, with two injections on consecutive days per week for 8 weeks. The patient's disease condition is monitored by means described further below. Three months after the last injection, if the patient is still suffering from the disease, the treatment regimen is repeated. The treatment regimen may be repeated until satisfactory result is obtained, *e.g.* a halt or delay in the progress of the disease, an -alleviation of the disease or a cure is obtained. Preferably, TF is formulated in an aluminum hydroxide adjuvant. The final 1 ml of the final TF formulation contains: 4 mg TF, 0.016 M AlPO₄ (or 0.5 mg Al³⁺), 0.14 M NaCl, 0.004 M CH₃COONa, 0.004 M KCl, pH 6.2.

Alternatively, the HIV infected patient or uninfected subject may be inoculated five months later, more preferably six months to two years later, and even more preferably eight months to one year later to enhance the patient's "immune memory". See Anderson ***et al., J. Infectious Diseases,*** **160(6)** : 960-969 (1989). Generally, infrequent immunizations with TF spaced at relatively long intervals is more preferred than frequent immunizations in eliciting maximum immune responses, and in eliciting a protective effect.

TF can be administered in various ways and to different classes of recipients. TF can also be used to vaccinate individuals who may or may not be at risk of exposure to HIV, and additionally, the vaccines are desirably administered to seropositive individuals and to individuals who have been previously exposed to HIV.

TF can be administered in combination with other antigens in a single inoculation "cocktail". TF can also be administered as in a series of inoculations administered over time. Such a series may include inoculation with the same or different preparations of HIV antigens or other vaccines.

The adequacy of the treatment parameters chosen, *e.g.* dose, schedule, adjuvant choice and the like, is determined by taking aliquots of serum from the patient and assaying for antibody and/or T cell titers during the course of the treatment program. T cell titer may be monitored by conventional methods. For example, T lymphocytes can be detected by E-rosette formation as described in Bach, J.F., ***Contemporary Topics in Immunology***, Vol. 2: Thymus Dependency, p. 189, Plenum Press, New York, 1973; Hoffman, T. & Kunkel, H.G., and Kaplan, M.E., *et al.*, both papers are in ***In Vitro Methods in Cell Mediated and Tumor Immunity***, B.R. Bloom & J.R. David eds., Academic Press, New York (1976). For example, the amount of T cell rosette formation may be assayed after the third but before the tenth week of TF treatment. An over sixty-five percent rosette formation indicates a good cell mediated immune response in the patient. A further indicator to monitor is aTF produced by the patient, this may be monitored by the two dimensional electrophoresis method described further below.

In addition, the clinical condition of the patient can be monitored for the desired effect, *e.g.* anti-infective effect. If inadequate anti-infective effect is achieved then the patient can be boosted with further TF treatment and the treatment parameters can be modified, *e.g.* to potentiate the immune response, such as by increasing the amount of TF and/or adjuvant, complexing TF with a carrier or conjugating it to an immunogenic protein, or varying the route of administration.

TF may optionally be administered along with other pharmacologic agents used to treat HIV infections such as AIDS and ARC. Examples of these pharmacologic agents are: AZT, antibiotics, immunomodulators such as interferon, anti-inflammatory agents and anti-tumor agents.

### Diagnostic Devices for Detecting HIV Infections

Another aspect of the invention presents diagnostic devices useful for ***in vitro*** detection of HIV infection. The device is designed to allow detection of the interaction between TF and aTF in a biological sample. More preferably, in the case where the biological sample is blood, serum, or plasma the device allows for the detection of precipitation pattern of TF in relation to β-, α₁-, and α₂- macroglobulins in the sample. Thus, preferably, the device contains a site for TF and a site for the test sample. Since a two-dimensional gel electrophoresis, as described in the following EXAMPLE 2 is preferred, the device most preferably consists of a gel with slots for containing TF and the test sample such as serum sample, respectively. The device is also preferably packaged with a kit with containers for the reagents necessary for conducting the test, such as buffer solution and TF. Preferably, the device has its own electrical source, such as a battery, to allow for a two-dimensional electrophoresis to be conducted. Otherwise, it is preferably designed such that it can be connected to outside electrical source.

Having described what the applicants believe their invention to be, the following examples are presented to illustrate the invention, and are not to be construed as limiting the scope of the invention.

### EXAMPLES

### EXAMPLE 1

### Isolation And Purification Of Thymus Factor

The following experiment shows the isolation, purification, and characterization of TF from calf thymus 4 hours after sacrifice.

### EXTRACTION(S) OF LYSINE-RICH HISTONE FRACTION FROM THYMUS CELLS WITH ENZYME DEGRADATION BY PEPSIN

All the buffers and solutions used in this section have been sterilized by filtration. If needed, the buffers' denoted pas were adjusted by 0.2 N NaOH or 0.1 N HCl. All the chemicals, including the distilled water, for the preparation of the buffers and solutions were USP grade.

The thymus tissue and its associated connective tissues were separated from a calf within 4 hours of its sacrifice. The tissues were washed with a solution containing 0.14 M NaCl, and 0.005 M EDTA-Na₃ at 4°C for 5 minutes. The wash solution was decanted and the tissues were washed a second time under the same conditions. After decantation of the wash solution, the tissues were weighed. The tissues were homogenized in 0.14 M NaCl, 0.005 M KCl, 0.005 M MgCl₂, 0.003 M CaCl₂, 0.15 M TRIS-HCl, pH 7.6, 0.25 M sucrose in a tissue homogenizer (Brinkman Polytron Homogenizer, Brinkman Instruments, Inc., Westbury, New York), at 4°C and at an rpm and for the duration of time recommended by the manufacturer of the homogenizer for removal of cell nuclei. The ratio of the tissue to the buffer was 1:4 (weight/weight).

The tissue homogenate was then filtered through gauze pad by vacuum. The filtrate was centrifuged at 1,000 g at 4°C for 90 minutes. The supernatant was discarded. The pellet was resuspended in 0.008 NaCl, 0.003 M CaCl₂, 0.003 M MgCl₂, 0.08 M NaH₂PO₄, 0.002 M TRIS-HCl, 0.25 M sucrose, pH 5.2. The ratio of the pellet to the buffer was 1:4 (weight/weight). The resuspension was homogenated in a beaker with a magnetic stirrer at 200 rpm, at 4°C for 5 minutes. The homogenate was then centrifuged at 3500 g, at 4°C, for 60 minutes. The supernatant was discarded. The pellet was resuspended in 0.014 M NaCl, 0.001 M CaCl₂, 0.002 M MgCl₂, 0.001 M EDTA-Na₃, 0.002 M TRIS-HCl, 0.25 M sucrose, pH 4.2 at a ratio of pellet to buffer of 1:4 (weight/weight). The resuspension was homogenated in a beaker with a magnetic stirrer at 200 rpm, at: 4°C for 5 minutes. The homogenate was then centrifuged at 8000 g, at 4°C for 60 minutes. The supernatant was discarded. The pellet was resuspended at a ratio of 1:4 (weight/weight) with a previously prepared buffer containing: 1 part volume/volume) Solution 1, 2 parts Solution 2, and 17 parts Buffer 4. Solution 1 consisted of: 10% sodium dodecyl sulfate in water/ethanol (at 55:45 v/v). Solution 2 consisted of 10% Tween™ 80 (in distilled water) . Buffer 4 consisted of: 0.011 M NaH₂PO₄ and 0.19 M Na₂HPO₄, pH 7.4.

The resuspension was homogenated in a beaker with a magnetic stirrer at 200 rpm, at 4°C for 15 minutes. The homogenate was then centrifuged at 12000 g, at 4°C for 60 minutes. The supernatant was discarded. The pellet was weighed and resuspended in 0.05 M Na₃C₆H₅O₇, 0.05 M CH₃COONa, 0.1 N HCl, pH 2.8 at a ratio of pellet to buffer of 1:4 (weight/weight). The resuspension was homogenized by tissue homogenizer at 1000 rpm, at 4°C for 1 minute.

Pepsin (catalog number P 7000, Sigma Chemical Company, St. Louis, Missouri) diluted in distilled water at 1:10000, with activity of 800-2500 units per mg protein, was added to the homogenate at a pepsin (powder) to pellet after homogenization weight ratio of 100:1.8. The mixture was placed in a beaker and stirred, under nitrogen atmosphere, with a magnetic stirrer at 45 rpm, at 4°C for 12 hours. The resulting mixture was then centrifuged at 12000 g, at 4°C for 60 minutes. The pellet was discarded. The supernatant was removed and precipitated with a solution consisting of saturated (NH₄)₂SO₄. One part of the supernatant was mixed with one part of the solution and stirred with a magnetic stirrer at 600 rpm for 6 hours at 4°C. The mixture was then centrifuged at 12000 g, at 4°C for 60 minutes. The supernatant was discarded. The pellet was dissolved in a solution containing a minimal quantity of 0.1 M NaCl, 0.1 M CH₃COONa, 0.02 M thiodiglycol. The resulting solution was dialyzed against 0.01 M NaCl, 0.01 M CH₃COONa, pH 6.4 until the ammonium sulfate was removed from the dialysate.

The protein in the dialysate was measured by the Lowry method. The solution was diluted to obtain 2 mg/ml of protein. The pH of the solution was then adjusted to 7.2 by 0.1 N NaOH. A solution of 0.2 M bromoacetic acid was prepared separately by dissolving bromoacetic acid in 10 ml or less of water and the pH was adjusted to 7.0 by 0.1 N NaOH. The resulting bromoacetic acid solution was added to the protein solution and the mixture was stirred by magnetic stirrer for 48 hours under nitrogen atmosphere. The pH of the mixture was maintained at 7.2 throughout the reaction. At the end of the reaction, the ammonium sulfate precipitation step was repeated up to the step of measuring the protein concentration by the Lowry method. The final solution was diluted or concentrated, as needed, to obtain 4 mg/ml of the protein TF from 200 g of calf thymus cells.

### CARBOXYMETHYL COLUMN CHROMATOGRAPHY

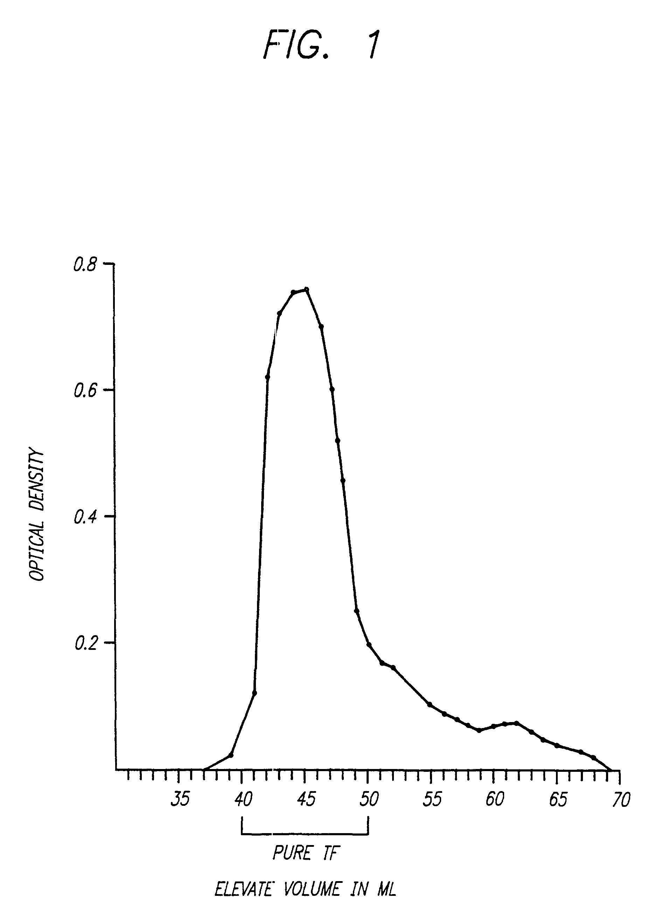

One ml of the above TF solution containing 4 mg of protein was applied to 0.9 x 14 cm carboxymethyl column equilibrated with 0.05 M sodium acetate. The column was washed and eluted by applying a linear gradient of sodium chloride in 0.05 M sodium acetate, pH 6.8. To create the gradient, siphon was used to connect two flasks of the same size and shape. One flask contained 50 ml of 0.05 sodium
acetate, pH 6.8 and the other flask contained 50 ml of 0.05 sodium acetate containing 0.5 M sodium chloride, pH 6.8. Only the flask containing 0.5 M sodium chloride was connected to the column. The gradient was from 0 to 0.5 m sodium chloride. The two flasks must be stirred vigorously to insure good mixing. Fractions of 1 ml each were collected. The eluate was examined by a flow spectrometer adjusted to 280 nm of absorbance reading. Eluate fraction 45 was determined to contain protein (see FIG. 1). The TF fraction was centrifuged at 10,000 rpm and two bands of precipitates were observed. Fraction 45 was collected and concentrated by ammonium sulfate precipitation.

The resulting concentrated fraction was further analyzed as follows.

### i) Determination of Molecular Weight

The molecular weight of the collected TF fraction was determined by silver stained 11% non-reducing SDS-PAGE (sodium dodecyl sulfate-polyacrylamide gel electrophoresis) using the Laemmli method {Laemmli, U.K., ***Nature***, 227:680 (1970)} and according to the instruction disclosed in Sigma Technical Bulletin MWS-877L (Sigma Chemical Company, St. Louis, Missouri). Rapid Silver Stain (RSK-1, Sigma Chemical Company) was used to stain the proteins. Low molecular weight standard (M 5630, Sigma Chemical Company) was used, it contained a mixture of five proteins (total protein concentration 1 ng/ml) : bovine serum albumin (66,000 molecular weight, MW) ; porcine heart fumarose (48,500 MW) ; bovine erythrocytes carbonic anhydrase (29,000 MW) ; bovine milk β-lactoglobulin (18,400 MW) ; and bovine milk α-lactalbumin (14,200 MW).

Two bands were observed on the gel. The larger band was unmethylated TF which constituted 98% of the TF fraction. The smaller band was methylated TF which constituted the remaining 2% of the TF fraction.

Based on the protein calibration curve, unmethylated TF was determined to have a molecular weight of about 35 Kilodalton (Kd) and methylated TF has a molecular weight of about 28 Kd.

The TF composition (including methylated and unmethylated TF) has a pH of between about 7.64 and 8.6 by pH titration. The isoelectric point of the TF composition is about 5.6 ± 0.1 as determined by immunoelectrical focusing.

### METHYLATION OF THE PROTEIN FOUND IN THE TF FRACTION

The TF was methylated to form a methylated TF preparation for use in the following EXAMPLES. The methylation was conducted as follows.

TF was mixed with CH₂BrCOOH in sufficient quantity to produce a final solution of 0.2 M CH₂BrCOOH. For example, 2.78 g CH₂BrCOOH was dissolved in 10 ml distilled water and added to 100 ml TF containing 700 mg TF (7 mg/ml). The pH of the mixture was adjusted to and maintained at 7.24 with 0.1 M in NaOH. The mixture was allowed to incubate from 6 to 8 hours. The TF protein in the resulting aqueous fraction was concentrated by ammonium sulfate precipitation, using techniques known in the art. To 10 ml of the methylated TF fraction was added an equal volume of saturated ammonium sulfate. The mixture was refrigerated 12 hours and then centrifuged 20,000 rpm for 60 minutes. The pellet was removed and dissolved in a final buffer containing 0.1 M NaCl, 0.1 M sodium citrate, 0.02 M thiodiglycol. The mixture was then dialyzed against the same buffer, to remove the ammonium sulfate, for 24 hours.

### EXAMPLE 2

### Diagnosis of HIV Infections

The methylated TF obtained from EXAMPLE 1 was used to determine whether a given human serum sample was from a patient infected with HIV. The experiment was conducted as follows:

The human serum samples in this Example were provided by AIDS Health Foundation, Los Angeles, California. The Foundation had tested the samples using commercially available enzyme-linked immunosorbent assay (ELISA) and Western blot which detected the patient's antibodies against the HIV. The ELISA was conducted first. If the ELISA indicated HIV infection (HIV-positive), Western blot was conducted as a confirmation test.

Using the present invention, the above samples were tested using two-dimensional electrophoresis. The equipment used was a submarine mini gel electrophoresis unit. (Catalog No. E 0638, Sigma Chemical Company). The unit contained a buffer chamber with a capacity for 800 ml of buffer. The unit was also equipped with a peristaltic pump for recycling the buffer. The power source had an output range of 20 to 240 V, 0 to 100 mA, with a constant current and voltage output.

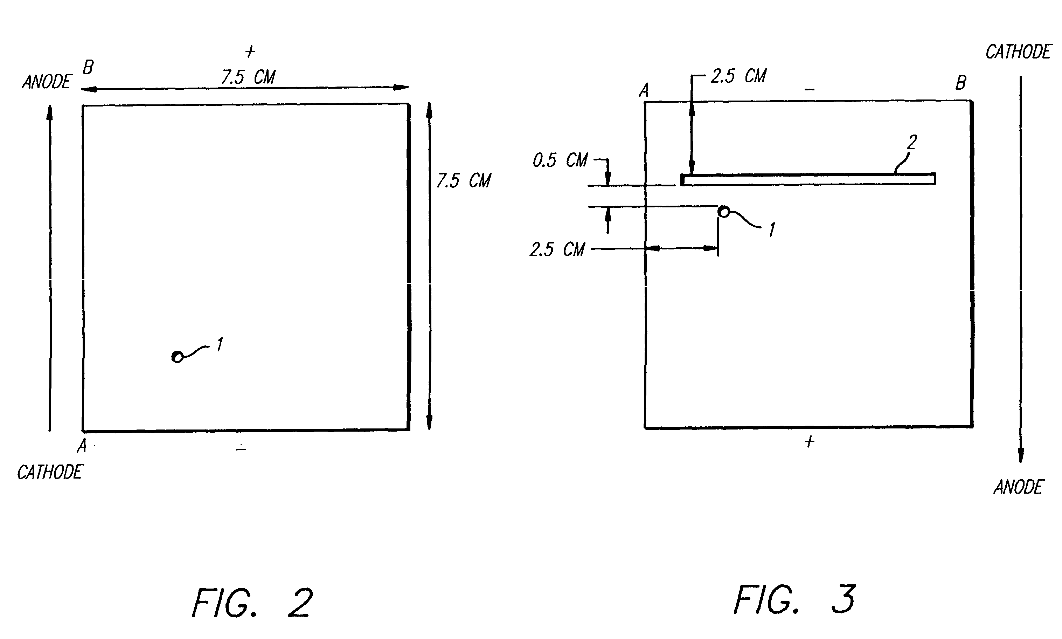

The gel was prepared with 1% agarose (Catalog No. A 4679, Sigma Chemical Company). The gel buffer consisted of: 0.0257 M TRIS-HCl, 0.009 M sodium borate decahydrate, 0.067 M glycine, 0.0034 M sodium acetate trihydrate, 0.015 M 2-mercaptoethanol and 0.015 M thiodiglycol, pH 7.64. 14.25 ml of the 1% melted agarose gel was applied to the electrophoretic gel plate. The solidified gel was 0.2 cm thick and at a dimension of 7.5 cm x 7.5 cm. The gel was overlaid with the same buffer. A well of 0.9 mm radius was punctuated in the gel using a syringe needle (with a bore of 0.9 mm in diameter) which delivered 5 *µ*l of the serum sample. For the first-dimension run, to separate the serum proteins, the voltage used was 9 V/cm, the current was 27 mA/cm², and the electricity was applied for 90 minutes at 14°C. FIG. 2 schematically presents the arrangement for the first-dimension gel, 1 denotes the well for the serum sample. For the second-dimension run, the gel plate was turned 90° and a 0.9 x 45 mm trough was removed from the gel from the side of the new cathode position, between the new cathode position and the serum hole and perpendicular to the cathode-anode line. FIG. 3 schematically presents the arrangement for the second-dimension gel, 2 denotes the trough. The trough was filled with 100 *µ*l of methylated TF of EXAMPLE 1. The same voltage and current was applied to the gel for 50 minutes at 14°C. Throughout the two- dimensional electrophoresis, the buffer was recirculated at 25 ml/min. After the last run, the power was turned off, and the gel was removed after a few seconds. The gel was then simultaneously fixed and stained, for 45 minutes, in a solution containing: 3 parts methanol, 1 part ethanol, 12% acetic acid and 0.1% bromophenol blue (Catalog No. B-8026, Sigma Chemical Company). The gel was destained by soaking it in distilled water for 3 to 4 hours and dried.

For molecular weight determination, a control two-dimensional electrophoresis was separately conducted under the same conditions but using the silver stain low molecular weight markers instead of serum and TF described in EXAMPLE 1.

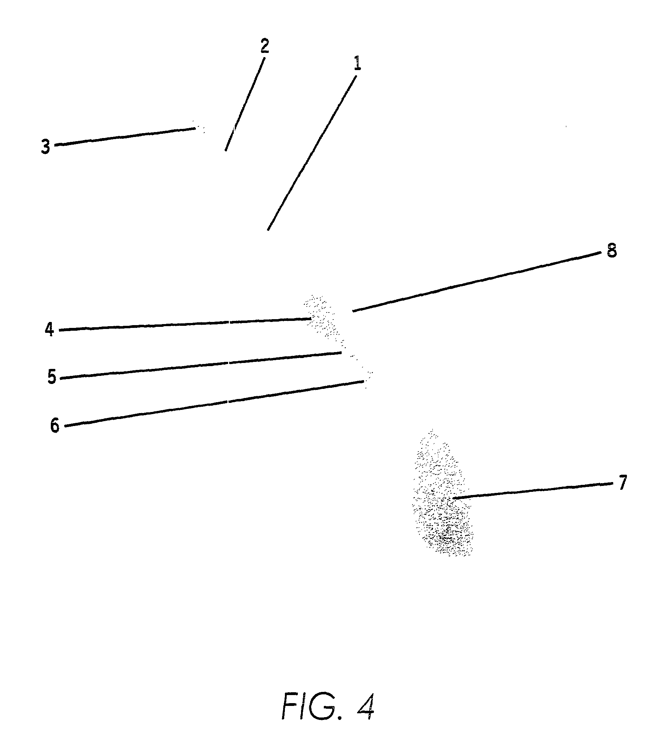

The positions of serum proteins on the two-dimensional electrophoretic gel were shown in FIG. 4. In FIG. 4, the immunoglobulin stain **3** appeared next to the serum well **1** containing the patient's serum sample, so did β-macroglobulin **4**, further from the serum well were α₂-macroglobulin **5**, α₁-macroglobulin **6**, and albumin **7**.

Precipitations of TF with aTF in the sera were detected by the appearance of fine opaque lines (precipitation spurs). The patterns of these precipitation spurs indicate whether the test subject has HIV infection:
A continuous precipitation spur **8** continuing alongside β- and α₁-macroglobulins, to alongside α₂- macroglobulins, as shown in FIG. 4, indicates that the test subject is not infected with HIV; a non-continuous (*i.e.* broken) precipitation spur alongside β-, *α*₁-, and α₂-macroglobulins, and/or spur(s) that are not alongside any or all of the macroglobulins indicate HIV infection.
Further, as the test subject's HIV infection (such as AIDS) progresses, the precipitates were less and thus the precipitation spurs became fainter.

The above observation suggests that aTF, found in healthy sera, was fragmented during HIV infection resulting in broken spurs of precipitation between TF and the fragmented aTF. The fragments were of smaller molecular weights than the whole aTF, thus they migrate further in the electric field. Thus the precipitation spurs were further from the TF trough. Without wishing to be bound by this hypothesis, it is hypothesized that the aTF would be fragmented into more parts when the AIDS, the HIV-related disease or the HIV infection is more advanced, thus there would be more broken precipitation spurs at greater distances from the groove containing TF. Thus, the pattern of precipitation is also indicative of the stage of the disease's progression.

Thus, the gels shown in the drawings are interpreted as follows:

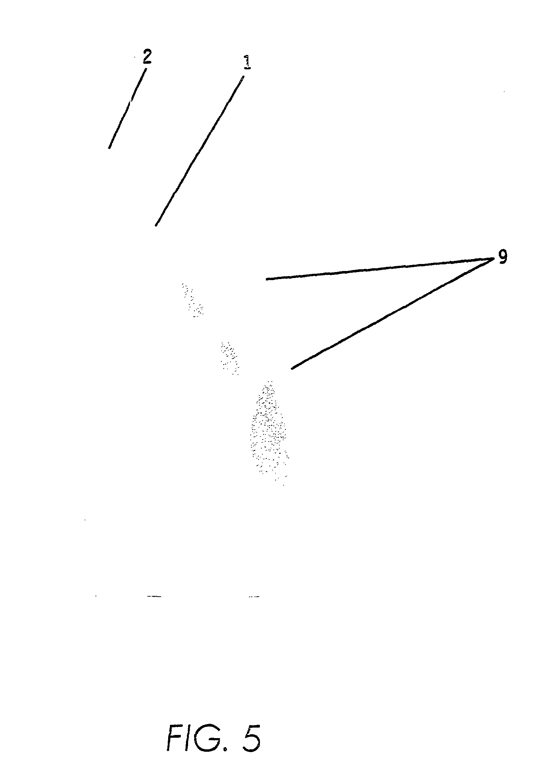

FIG. 5 shows broken precipitation spurs 9 which indicate that the subject was HIV-positive. This subject's serum was taken and tested HIV-positive by ELISA and the test of the present invention. At the time the serum was taken, the subject did not show any clinical symptoms of AIDS.

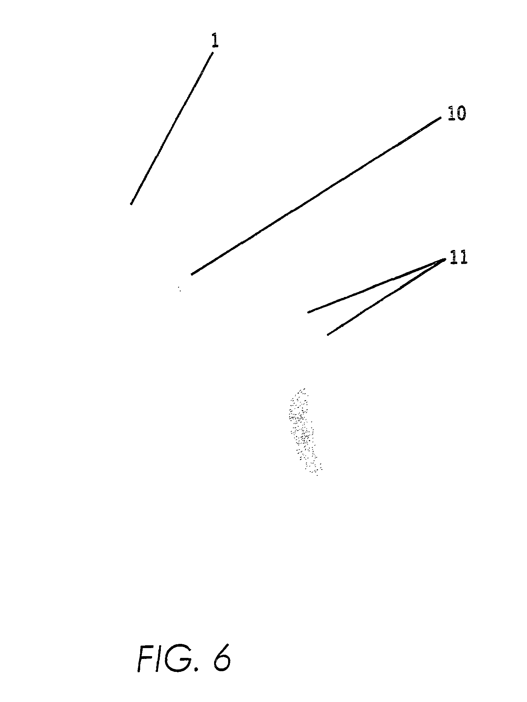

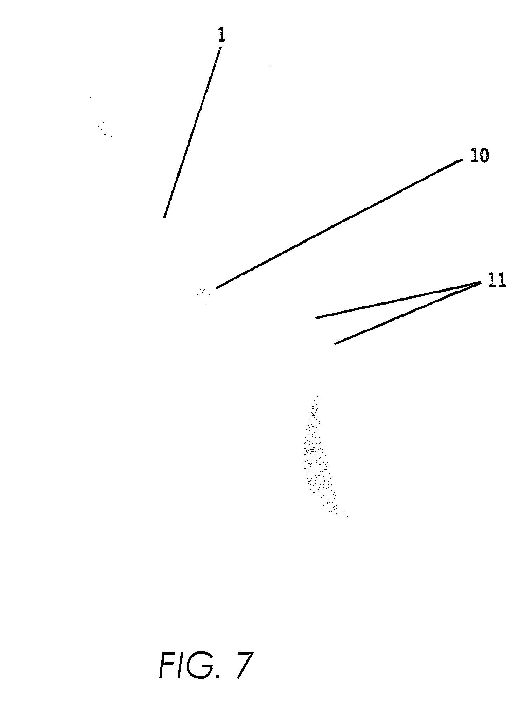

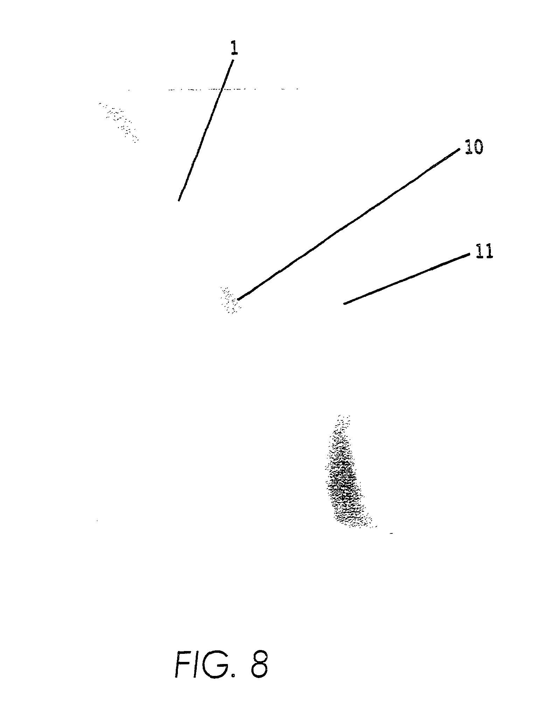

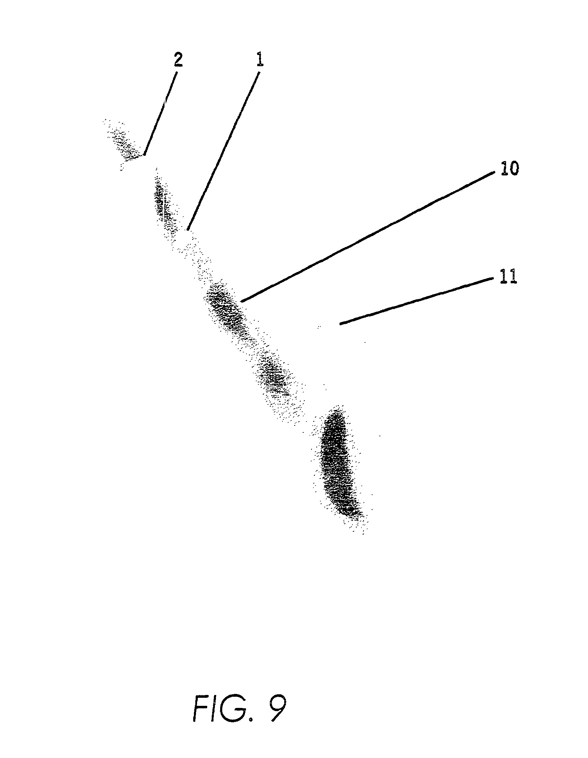

FIGs. 6 to 9 show position 10, where β-macroglobulin was located and where a healthy subject's precipitation spur would be adjacent to, and the spur would be a continuous spur spanning alongside the locations of β-, α₁-, and α₂- macroglobulins (as in FIG. 4). In contrast, in FIGs. 6 to 9, the subjects' precipitation spurs 11 were broken (*i.e.* not continuous alongside the locations of β-, α₁-, and α₂- macroglobulins) and below position 10. The positions of the test subject's precipitation spurs indicated that they were of lesser molecular weight than the precipitation spurs of a healthy subject. The broken fragments and spurs and their lesser molecular weights indicated that the test subjects were HIV-positive. Between three to six months before the present tests, these subjects were tested HIV-positive by ELISA. The present test used the sera taken at the same time as that for the ELISA test. At the time of the present tests, the subjects exhibited clinical symptoms of AIDS. The severity of the disease among the subjects were in the order of subject of FIG. 6 (most severe case of AIDS), FIG. 7, FIG. 8 and FIG. 9 (least severe case of AIDS). The clinically observed severity of the disease was consistent with the patterns of the precipitations in the figures shown. First, gels with precipitation spurs that are further away in distance, from the location of spurs expected of a healthy subject, indicate smaller fragments of aTF and thus a more severe case of AIDS than gels with precipitation spurs that are closer to the expected location of the spurs of a healthy patient. For example, this distance can be determined based on the relative distance of the first precipitation spur, *i.e.* the spur closest in vertical distance to the serum hole **1**, from the expected spur of a healthy subject should be. The further the relative distance, in both the horizontal and vertical directions from the location of the health subject's spur, the more severe the disease. Second, if the above factors cannot distinguish between the gels of two AIDS subjects, the disease is less severe for the subject who has a gel showing spur(s) that are more intense and lesser in number. Lesser number of precipitation spurs and a heavier precipitation indicate aTF that is less fragmented and at a higher level, and therefore, a less severe case of AIDS.

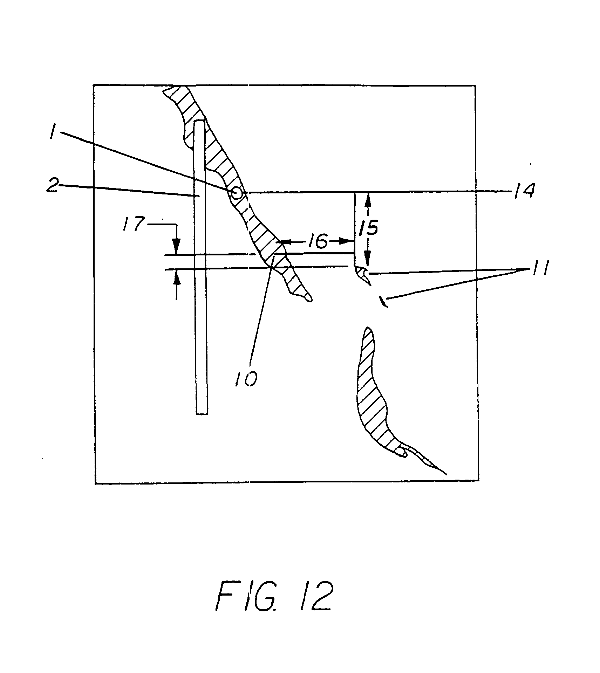

An example of the application of the first analysis is applied to analyzing FIGs. 6 to 10. In order to normalize the comparison of these figures, a fixed point for all the figures, in this case, serum hole **1** is chosen as the baseline **14**. The first precipitation spur is the one closest in vertical distance **15** from the baseline **14** (see FIG. 12). Since a healthy subject's precipitation spur would stretch from adjacent to the locations of β-and α₁-macroglobulins, to the location of *α*₂-macroglobulins (as in FIG. 4), one can measure the relative distance from either the location of β-, α₁-, or α₂- macroglobulins. In this case, the location of β-macroglobulin **10** was chosen. Both the vertical **17** and horizontal **16** distances of the spur from the location of β-macroglobulin **10** (see FIG. 12) were measured and compared for these series of figures. Subjects whose sera produced gels with greater -vertical **17** and/or horizontal **16** distances of the spur from the location of β-macroglobulin **10** have more severe case of the disease than those with lesser distances. The comparison showed the following: FIG. 6 (most severe case of AIDS), FIG. 7, FIG. 8 and FIG. 9 (least severe case of AIDS). This is an example of the application of the first analysis. One skilled in the art will realize that the baseline can be another position that is standardized for all the gel photographs, and the relative distances can be measured from another point that is located in the spread of the serum, as long as these points are consistent for all the photographs being compared and consistently used to measure the relative distances. Other variations that do not depart from the spirit of the first analysis can be used.

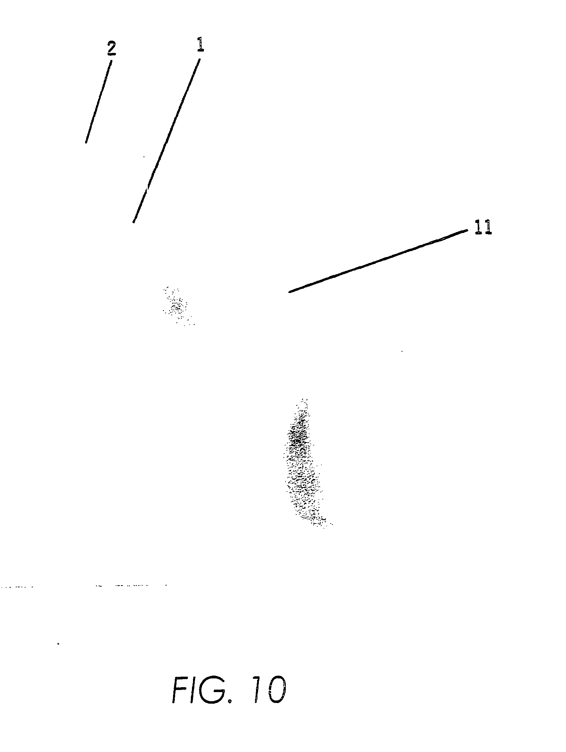

FIG. 10 is significant in that at the time the serum for this test was taken, the subject tested HIV-negative on the ELISA. However, the test of the present invention indicated that the subject was HIV-positive as shown by broken precipitation spur **11**. Two months after the serum for the test was drawn, the subject tested HIV-positive on the ELISA. Thus, the figure confirms that the test of the present invention is capable of an earlier detection of HIV infection than the conventional ELISA.

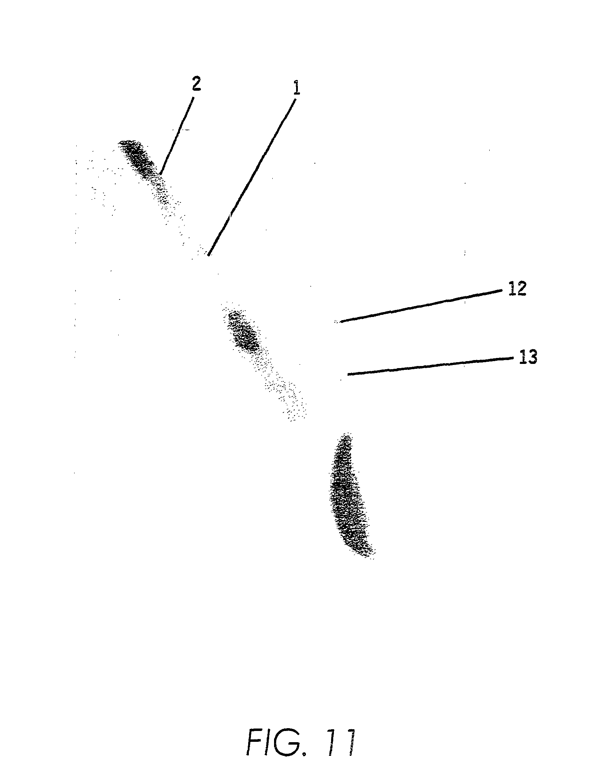

FIG. 11 is significant in that the serum sample was taken after a clinically diagnosed AIDS subject had been transfused with blood from a healthy donor. The precipitation spur caused by the donor's serum is indicated by the arrow **12** at the location of higher molecular weight, which tends to show that the initial small amount of fragmentation of whole aTF from the healthy donor, and thus the location of the spur was closer to the molecular weight expected of a healthy subject and more indicative of an earlier stage of infection, as shown by the relatively similar locations of this spur with the spur in FIG. 10. The precipitation spur caused by the serum of the AIDS subject is indicated by the arrow **13**, at a location of lower molecular weight, which indicates fragmented and thus smaller aTF from the patient and HIV-infection.

The two-dimensional gel electrophoresis shown herein can be mass-produced as an ***in vitro*** device for testing of biological fluid samples for the presence of HIV infection from the test subjects. The gels can be dried for easy packaging and transportation. The packaged gel may contain a preformed well for samples, and a trough for TF. The TF can be stored in a container and sold separately or together with the gel as a kit. Additionally, the kit may also contain containers for the electrophoretic buffer, the stains and/or molecular weight standards. For the control, the kit may further contain containers with serum sample(s) from healthy subject (s).

Besides the above specified assay, included in the present invention are variations of the above assay and any assay which allows for the determination of the precipitation pattern of a subject's biological fluid sample with aTF, preferably in relation to β-, α-, and α₂-macroglobulins. Kits containing reagents and materials needed for these assays are also included in the present invention.

### EXAMPLE 3

This example describes a study in which an AIDS patient (also referred to as "test subject" in this EXAMPLE 3) was therapeutically treated with the TF composition of the present invention. The patient did not receive any other treatments or drugs during the study.

The patient was a 27 year old White homosexual male, weighing 138 pounds at the beginning of the trial. Eighteen months ago, the patient's serum tested positive for HIV antibodies. The patient was also diagnosed as HIV positive based on his clinical symptoms, p24 antigen count, CD4⁺ and CD8⁺ on cell counts in absolute numbers (cells/*µ*l) and lymphocyte subset percentages, as shown in Table 1 below for the tests conducted on the patient's blood sample taken on April 12, 1995, two weeks before he started his TF treatment. The patient was not suffering from AIDS before and during the TF treatment.

The treatment started on April 27, 1995. The patient received 14 mg of TF per week in two intramuscular injections, one injection on Tuesday and another on Wednesday. Each injection contained 7 mg of TF (in 2 ml of a formulation containing 3.5 mg/ml of TF). The TF was purified as described in Example 1 above and the sterilized TF formulation contained: 3.5 mg/ml methylated TF, 8.1 mg/ml sodium chloride, 1.9 mg/ml sodium acetate, 2.6 mg/ml sodium tri-phosphate, 2.1 mg/ml aluminum chloride, pH 6.2.

Table 1 presents the results of the experiment. The dates are the dates on which blood samples were taken from the patient. The tests were conducted by Unilab Corporation (Tarzana, California) , using generally accepted clinical assays.

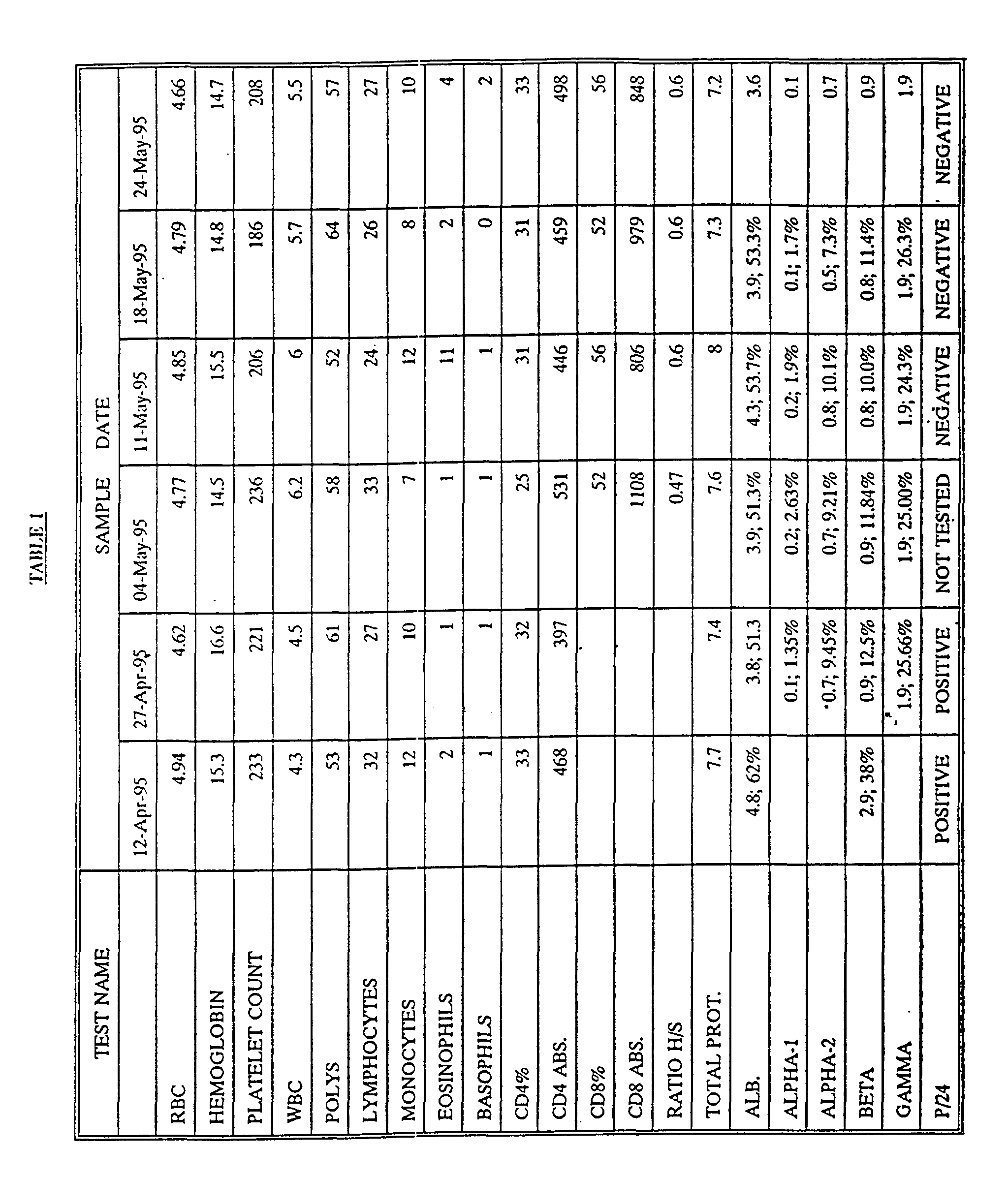

The tests are as follows:
"RBC" and "WBC" denotes red and white blood cell counts, respectively (x 10³/mm³).
"HEMOGLOBIN" and "PLATELET COUNT" denote the haemoglobin counts (in g/dL) and platelets counts (x 10³/mm³), respectively.
"POLYS" denotes neutrophil cell counts measured as a percentage of white blood cell counts.

The "LYMPHOCYTES", "MONOCYTES", "EOSINOPHILS", and "BASOPHILS" counts were each measured as a percentage of white blood cell counts. The above complete blood counts were determined using hematology analyzer.

"CD4%" and "CD8%" denote the lymphocyte subset percentages of CD4⁺ and CD8⁺ cells, respectively. "CD4 ABS" and "CD8 ABS" denote the absolute cell numbers (in cells/*µ*l) of CD4⁺ and CD8⁺ cells, respectively. The CD4⁺ and CD8⁺ cell counts were determined by flow cytometry.

"RATIO H/S" denotes the ratio of the Helper versus the Suppressor cells and was obtained by dividing the number of "CD4 ABS" with the number for "CD8 ABS".

"TOTAL PROT." denotes the total blood serum protein in g/dL in the patient's sample, as determined by colorimetric method using Olympus AU 5000 instrument (Olympus Co., Ltd., Tokyo, Japan).

"ALB." denotes albumin; "ALPHA-1" denotes alpha-1 globulin; "ALPHA-2" denotes alpha-2 globulin; "BETA" denotes beta-globulin; "GAMMA" denotes gamma-globulin. These serum proteins were determined by serum protein electrophoresis. For each column of a specific serum protein, the number to the left is in g/dL, and the number to the right indicates the percentage distribution of the specific serum globulin among all the serum globulins.

"P/24" denotes p24 viral core antigen which was determined by p24 antigen EIA test kit (Abbott Laboratories, Abbott Park, Illinois).

Further, the blood samples collected on the time points specified in Table 1 also tested positive for HIV-1 antibodies as determined by HIV ELISA test kit of Organon Technica (Raleigh, North Carolina). The patient gained 7 pounds in weight between his first TF injection and up to May 24, 1995.

The above tests show a trend of increasing CD4⁺ cell counts and decreasing CD8⁺ cell counts after the start of the TF treatment. Additionally, the p24 antigen tests showed negative results after the start of the TF treatment, though the test subject tested positive before and at the start of the TF treatment. The HIV antibody assays remained positive as residual antibodies remained in the test subject. These test results tend to suggest that the TF treatment was effective in combating HIV-infection and the progression of the disease caused by HIV.

In addition to the above tests conducted by Unilab Corporation, applicant also conducted the HIV diagnostic tests, two-dimensional electrophoresis, described in EXAMPLE 2, above, on the serum samples collected at the time points stated in Table 1.

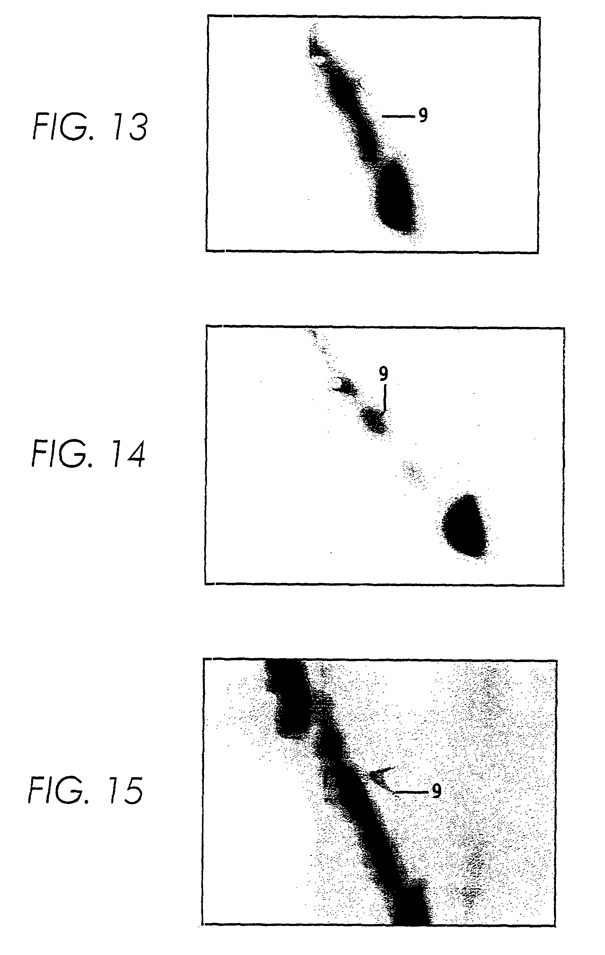

FIGs. 13 and 14 are photographs of two-dimensional electrophoretic gels of serum samples from two controls: a human subject whose serum sample tested negative for HIV antibodies in the ELISA test of Organon Technica, and a human subject whose serum sample tested positive in the same ELISA test. It can be seen that in FIG. 13, the precipitation spur **9** is a continuous spur spanning alongside the locations of β-, α₁-, and α₂-macroglobulins, which indicate a healthy HIV negative subject. In contrast, the precipitation spur **9** in FIG. 14 is discontinuous, indicating HIV infection in the subject.

FIGs. 15 to 19 present the resulting photographs of the two-dimensional electrophoretic gel tests for the test subject, on the serum samples collected on May 11, May 18, May 24 and May 31, 1995, respectively.

As shown in FIG. 15, after a week of receiving the above TF treatment, on May 4, 1995 the test subject's serum sample tested negative for HIV infection, as indicated by the continuous precipitation spur **9** alongside the locations of β-, α₁-, and α₂-macroglobulins.

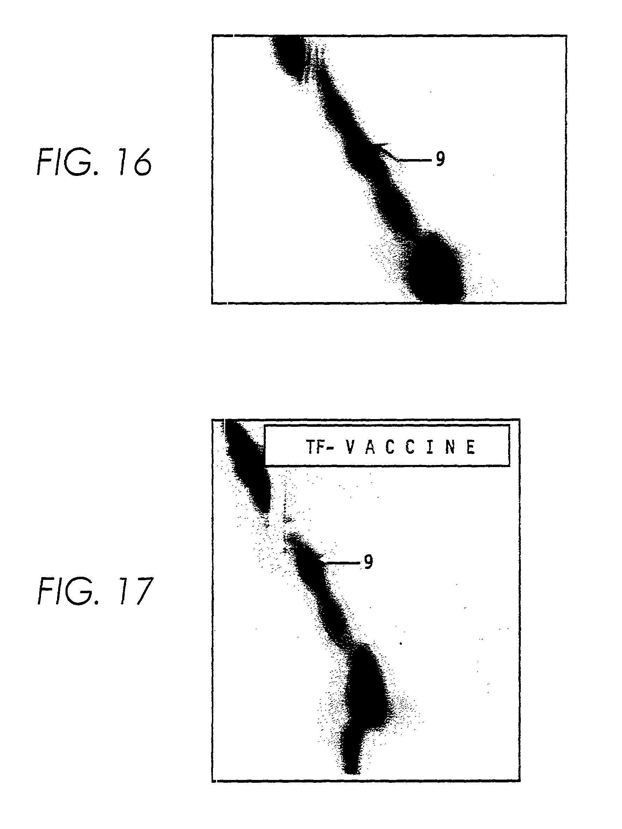

As shown in FIG. 16, in the second week of treatment, on May 11, 1995, the test subject's serum formed a continuous precipitation spur **9** alongside the locations of β- and α₁- macroglobulins, which is a more continuous precipitate spur than that observed for the control HIV-positive patient of FIG. 14.

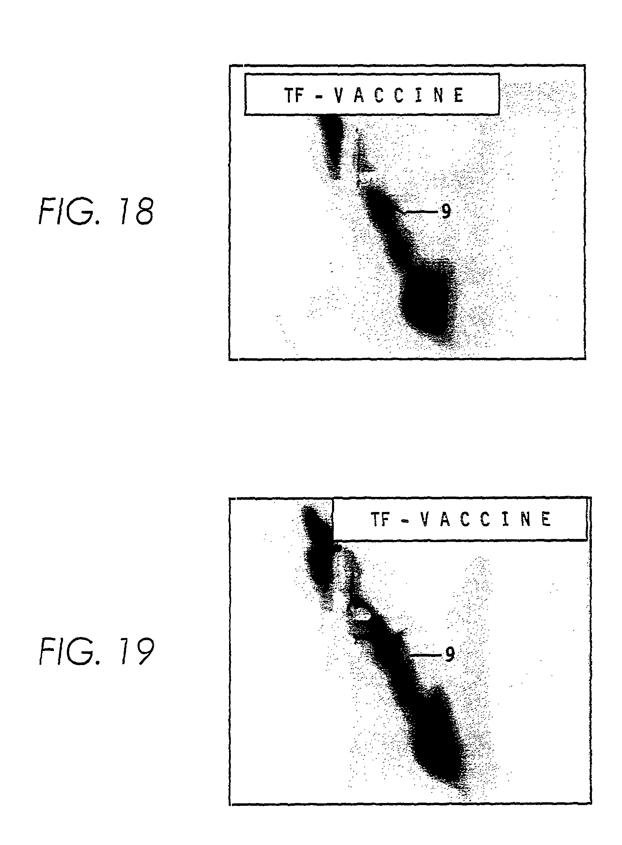

As shown in FIGs. 17 and 18, in the third and fourth weeks of treatment, on May 18 and 24, 1995, the test subject's serum formed a less continuous precipitation spur **9**. However, in the fifth week, on May 31, 1995, a continuous precipitation spur **9** is again observed alongside the locations of β-, *α* ₁-, and *α*₂- macroglobulins (as shown in FIG. 19), indicating HIV-negative status. The two-dimensional electrophoretic tests tend to suggest that the TF treatments of the present invention is effective against HIV-infection and/or disease progression.

### EXAMPLE 4

This Example provides further data regarding six human patients who were therapeutically treated with the TF composition of the present invention. These patients did not receive any other treatments or drugs during and after the six weeks of treatment period. The TABLES below also show patients' data at nine months (for Patient Nos. 1-5), and two months (for Patient No. 6) after the end of the treatment period.

For six consecutive weeks, each patient received 14 mg of TF per week in two intramuscular (IM) injections, one injection on Tuesday and another on Wednesday per week. Each injection contained 7 mg of TF (in 2 ml of a formulation containing 3.5 mg/ml of TF). The TF was purified as described in EXAMPLE 1 above and the sterilized TF formulation contained: 3.5 mg/ml methylated TF, 8.1 mg/ml sodium chloride, 1.9 mg/ml sodium acetate, 2.6 mg/ml sodium tri-phosphate, 2.1 mg/ml aluminum chloride, pH 6.2.

TABLES 2 to 7 present the results of the study for each patient. The tests were conducted by laboratories using generally accepted clinical assays. In the TABLES, "HIV-Ab, ELISA" denotes HIV-1 antibodies as determined by ELISA. "CD4 abs." and "CD8 abs." denote the absolute cell numbers (in cells/*µ* l) of CD4 and CD8 cells, respectively. "p24 Ag" denotes p24 viral core antigen which was determined by p24 antigen immune complex disruption (ICD) EIA; this EIA for HIV-1 antigen detects p24 core protein after disruption of immune complexes. "HIV-1 RNA, PCR" denotes quantitative estimate of number of copies of HIV-1 ribonucleic acid (RNA) per ml of plasma tested by polymerase chain reaction (PCR) amplification. Increases/decreases in the number of copies of viral RNA detected are associated with increases/decreases in plasma viremia. "HIV-1 Plasma Culture" denotes HIV-1 titers found in plasma and peripheral blood mononuclear cell cultures. "HIV-1 Plasma Quantitative" denotes HIV-1 viral infectivity expressed as infectious unit per ml (IU/ml). "Poly Cells" denotes white blood cell counts in absolute numbers (cells/*µ*l). The α-1 macroglobulin (denoted as "alpha-1 Macro"), α-2 macroglobulin (denoted as "alpha-2 Macro"), and gamma-immunoglobulin ("IgG") were determined by serum protein electrophoresis. The number (with %) indicated is the percentage distribution of the specific serum globulin among all the serum globulins. In TABLE 7 "IgA" and "IgM" denote immunoglobulins A and M, respectively. In TABLE 7, the IgG, IgA and IgM are expressed in mg/DL serum. "HLA-DR" denotes a product of Human Major Histocompatibility complex located on chromosome 6.

Further definitions as used in TABLES 2-7 are:
- inc.: normal level for a healthy person;
- pos.: positive;
- +: positive, increased positive level is denoted by additional "+";
- neg.: negative;
not tested;
- nt: not tested;
- x: multiple of the normal level for a healthy person, ***e.g.,*** "2x" denotes twice the normal level.

The following describes in detail the conditions of each patients.

### Patient No. 1

The following TABLE 2 summarizes the results of clinical tests conducted on Patient No. 1.

At the start of treatment, Patient No. 1 (a 21-year old female) had full blown AIDS with cerebral damage and mental disorders, a CD4 count of 36, and a life expectancy in the range of six-twelve months. Her CD4 count increased from 36 (at the start of treatment) to 108 (nine months after the end of the treatment period). Beginning in the fourth week of treatment, the p24 core antigen was negative, ***i.e.***, at less that 5 *µ*g/ml. This negative p24 core antigen reading indicates lack of viral activity as of the fourth week of treatment. The plasma HIV-1 culture tested negative as of the fourth month of treatment. The negative plasma HIV-culture reading indicates that this patient's T-cells isolated from plasma were non-infective when mixed with T-cells of uninfected control blood in the ***in vitro*** assay -- *i.e.,* negative plasma culture. In contrast, positive plasma culture was produced when T-cells from control HIV-1 infected blood were mixed with uninfected control blood in the ***in vitro*** assay. Increases in IgG, α-1 and α-2 macroglobulin activities were observed during and after TF treatment. Nine months after treatment, the patient no longer had full blown AIDS (***e.g.***, no opportunistic diseases) , though she was still HIV positive (***i.e.***, tested positive for antibodies against HIV in an ELISA).

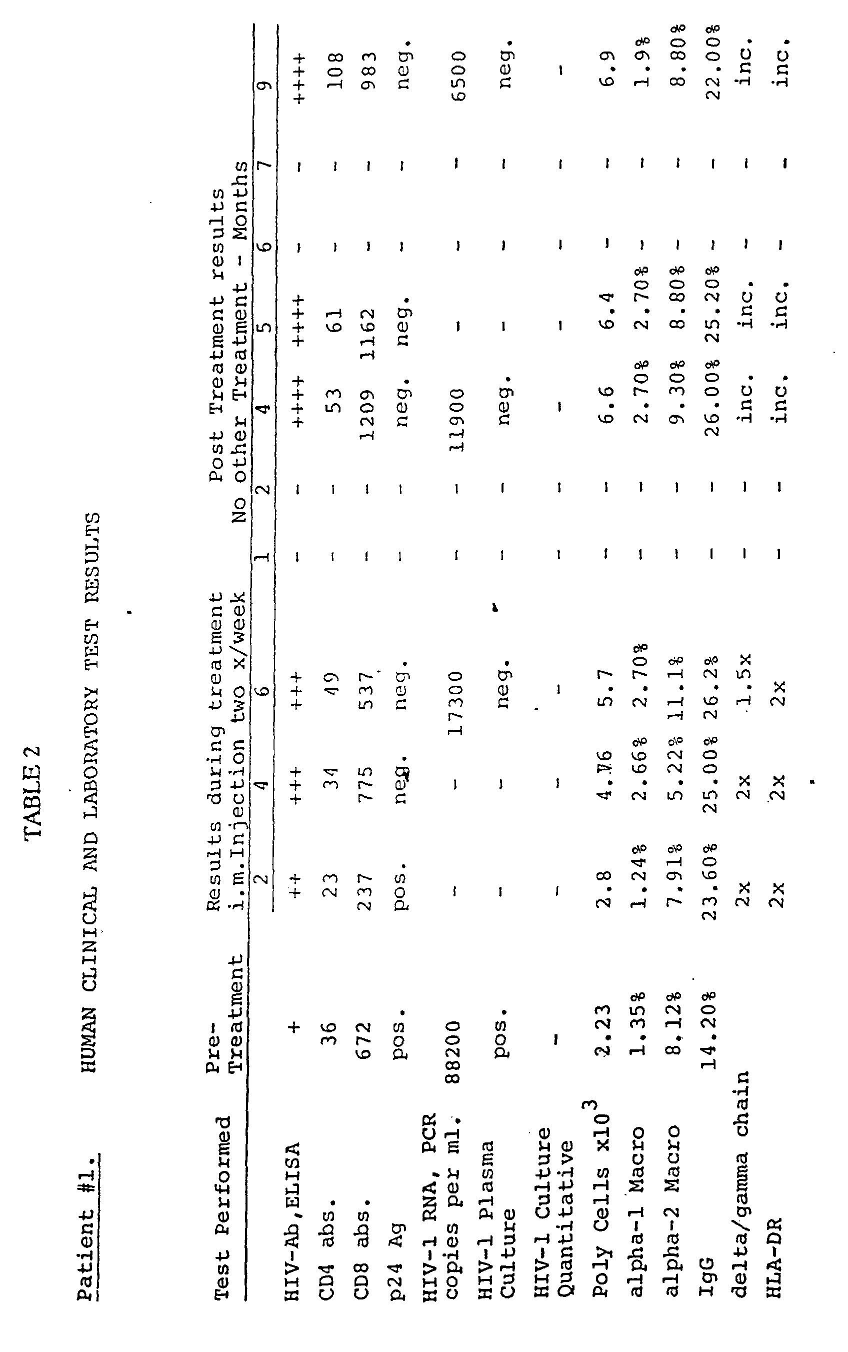

### Patient No. 2

The following TABLE 3 summarizes the results of clinical tests conducted on Patient No. 2.

At the start of the treatment, Patient No. 2 (a 30-year old male) was HIV positive (***i.e.***, tested positive for antibodies against HIV in an ELISA), however he did not suffer from all the symptoms of AIDS. His CD4 cell counts increased from 193 (before TF treatment) to 305 (nine months from the end of treatment) . His p24 core antigen remained negative before, during and after TF treatment -- indicating low viral activity. His plasma culture was converted to negative at fourth month after treatment, indicating non-infectivity of the his plasma in the ***in vitro*** assay. Increase in his humoral immunity was indicated by increases in the IgG, α-1 and α-2 macroglobulin levels. At nine months after the end of the treatment, the patient still did not show any sign of AIDS.

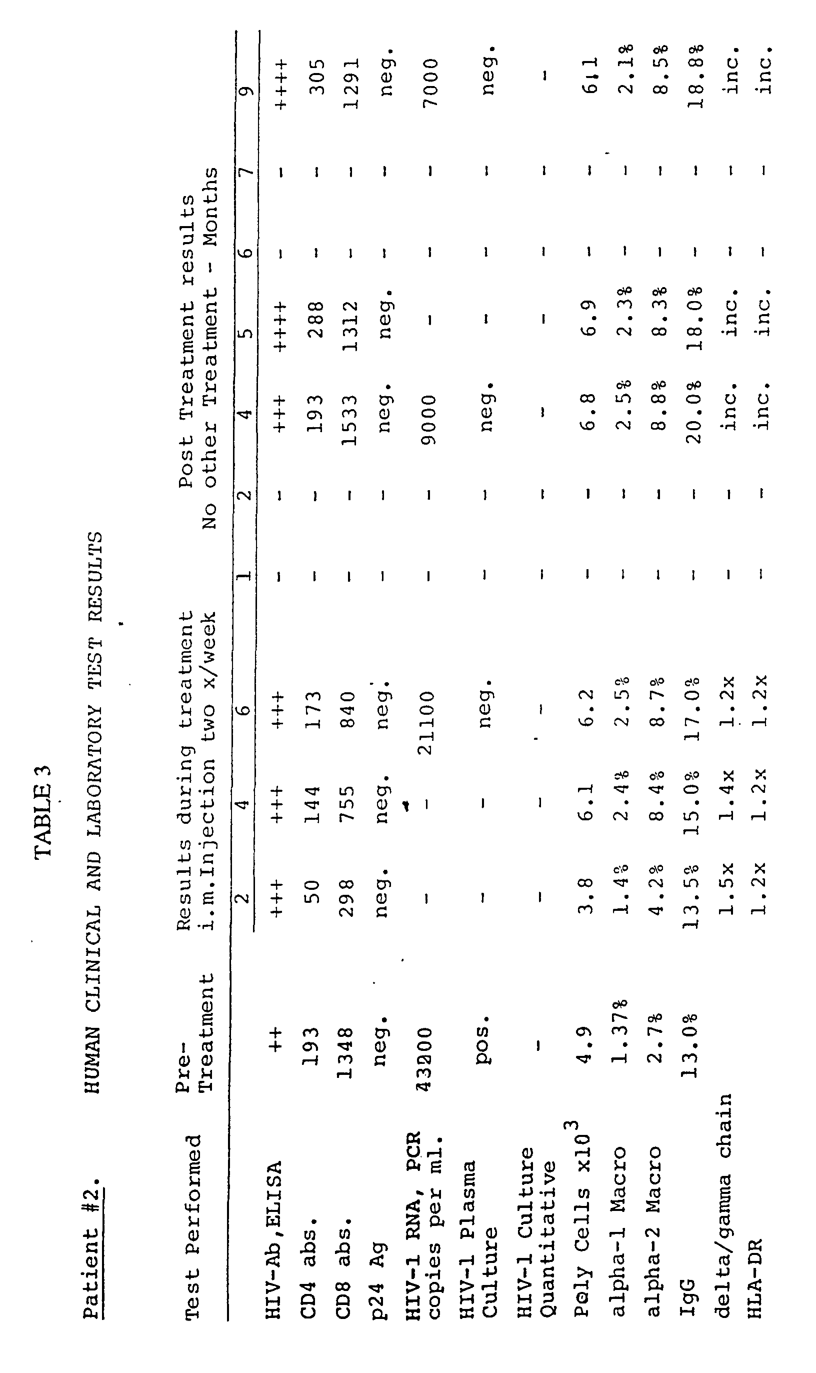

### Patient No. 3

The following TABLE 4 summarizes the results of clinical tests conducted on Patient No. 3.

At the start of the treatment, Patient No. 3 (a 24-year old female) was HIV positive (***i.e.***, tested positive for anitbodies against HIV in an ELISA), however she did not suffer from all the symptoms of AIDS. CD4 cell counts increased from 404 (pretreatment) to 636 (nine months from the end of treatment). Her p24 core antigen test was negative throughout the study. Her plasma culture converted to negative at fourth month after treatment. The increase in humoral immunity of the patient was indicated by increases in her IgG, α-1 and α-2 macroglobulins. Nine months after the end of the treatment, the patient did not show any sign of AIDS.

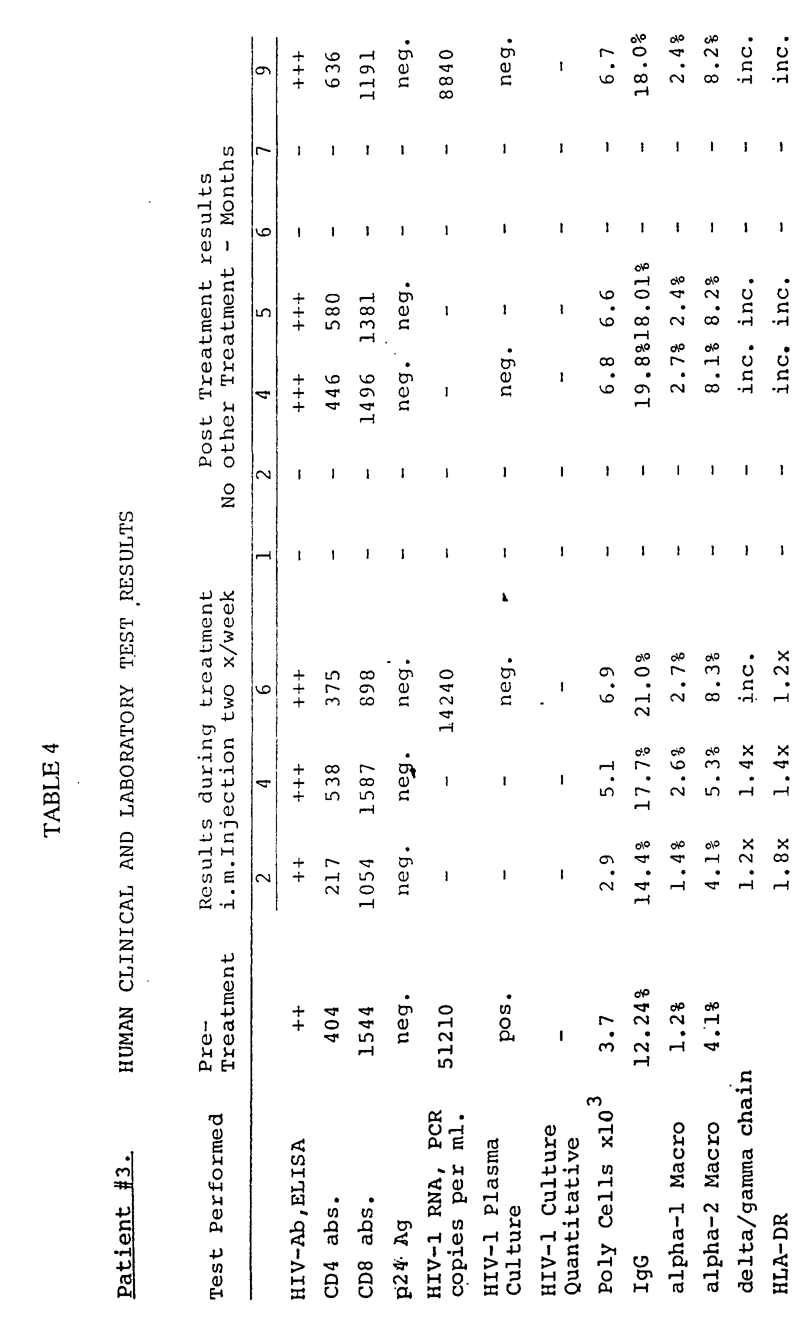

### Patient No. 4

The following TABLE 5 summarizes the results of clinical tests conducted on Patient No. 4.

Before treatment, the patient (a 27-year old male) was HIV positive (***i.e.***, tested positive for anitbodies against HIV in an ELISA), and had full blown AIDS symptoms (Grade 3, with opportunistic infections in the throat and upper part of the lung). His CD4 cell counts increased from 389 (before treatment) to 599 (nine months from the end of treatment). His p24 core antigen converted to negative after four weeks of treatment and remained negative. His HIV plasma infectivity was converted to non-infective after six weeks of treatment and remained non-infective. The increased in humoral immunity of the patient was indicated by the increase in his IgG, α-1 and α-2 macroglobulins. Twelve months after the end of the treatment, the patient did not show any sign of AIDS.

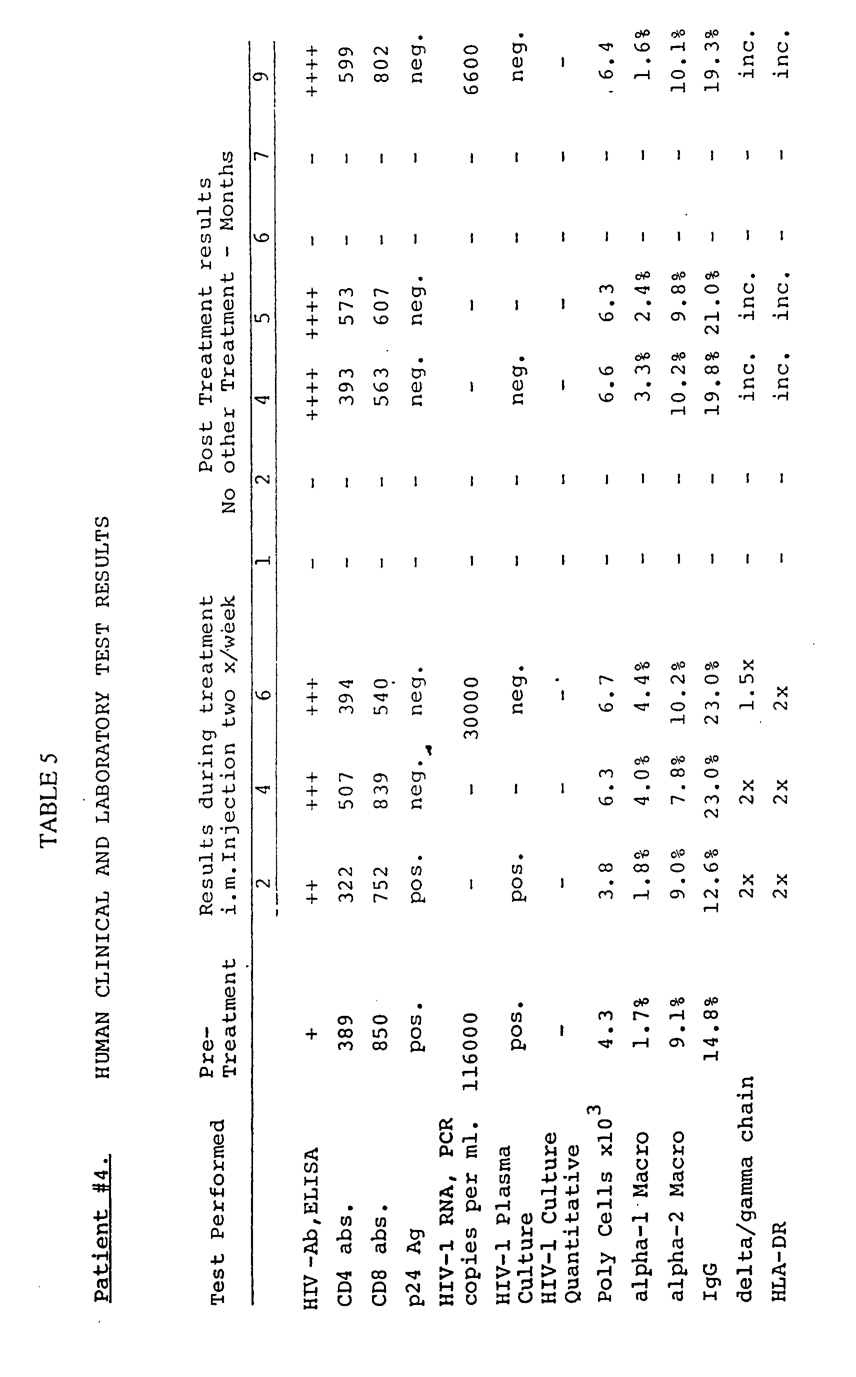

### Patient No. 5

This patient is the same as the one in **EXAMPLE 3**, above.

The following TABLE 6 summarizes the results of clinical tests conducted on Patient No. 5.

Before the treatment, the patient was HIV positive (***i.e.***, tested positive for anitbodies against HIV in an ELISA), suffering from pneumonia, vomiting, and diarrhea without blood in the excrement. However, he did not have full blown AIDS. His CD4 cell count remained constant after treatment. His p24 core antigen was converted to negative during the fourth week of treatment and remained so by seven months after treatment. The HIV-1, RNA, PCR showed that the number of viral copies had been maintained at about 6,000 - 7,000 copies/ml. His plasma culture was non-infective after treatment. His immune system produced increased of IgG, α-1 and α-2 macroglobulin. He had gained 10-12 pounds of weight. Ten months after the end of the treatment, he did not show any sign of AIDS.

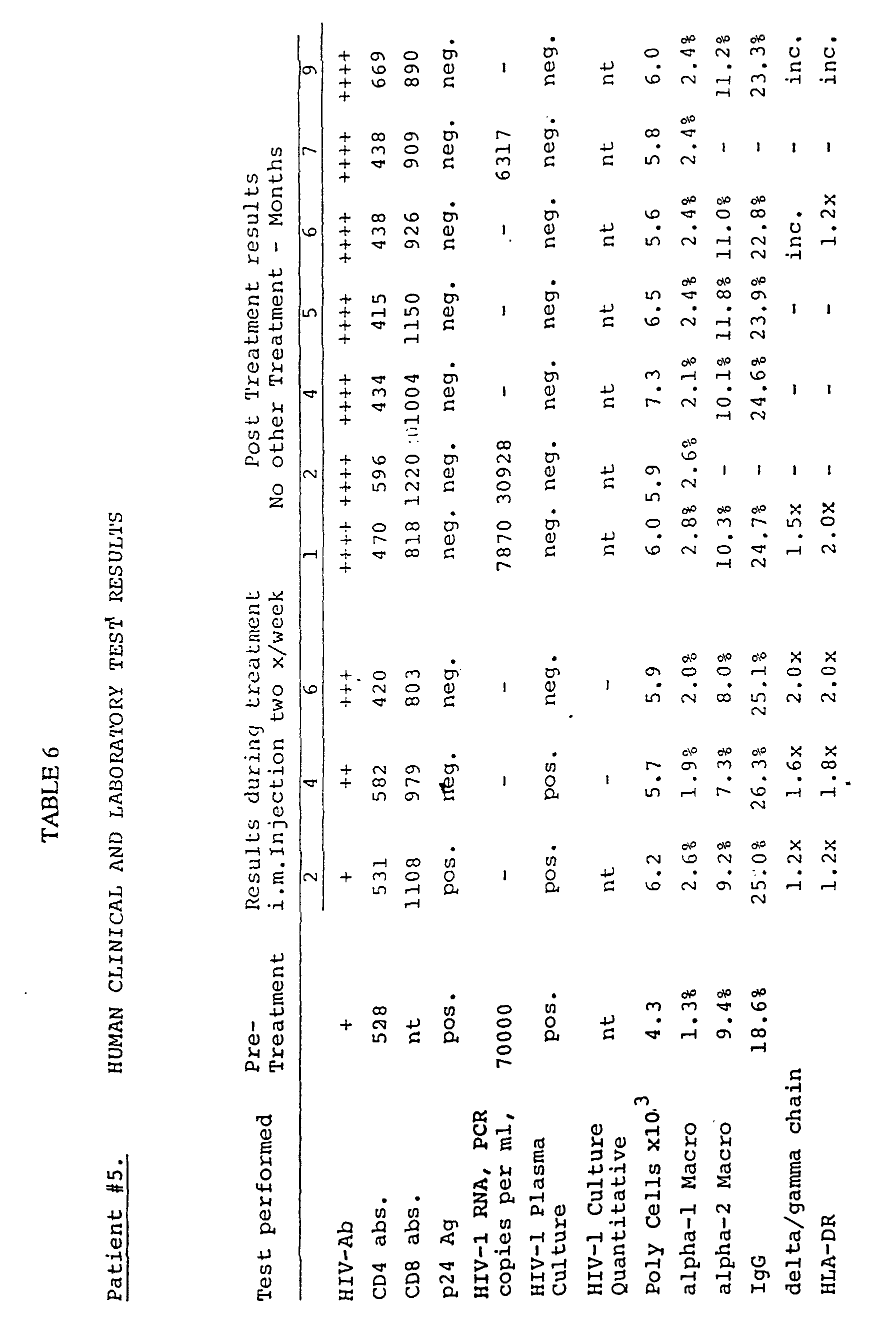

### Patient No. 6

The following TABLE 7 summarizes the results of clinical tests conducted on Patient No. 6.

At the start of the treatment period, the patient had full blown AIDS (at a grade between 3 and 4, suffering from ***e.g.***, retinitis, cytomegalovirus infection, fungal infection in the throat, lung, trachea, and bronchi, and bedridden). His CD4 count was slightly increased after two months of treatment. His p24 core antigen converted to negative after four weeks of treatment and remained negative. His plasma culture was positive before treatment and was converted to negative and non-infective after treatment. Generally, the peripheral blood cell cultures are positive in a patient with CD4 count of less than 100 and viral loads of 5x10⁵. However, in the case of Patient No. 6, no HIV was detected in the peripheral blood cells at one and two months of treatment. Significantly, the viral load of the patient had decreased from 5x10⁵ to 3x10⁴ copies of viral RNA. This is close to a 1.5 log decrease in viral RNA. Patient No. 6 had also gained 15 pounds of weight since the start of treatment. Three months after the end of treatment, the patient did not have AIDS.

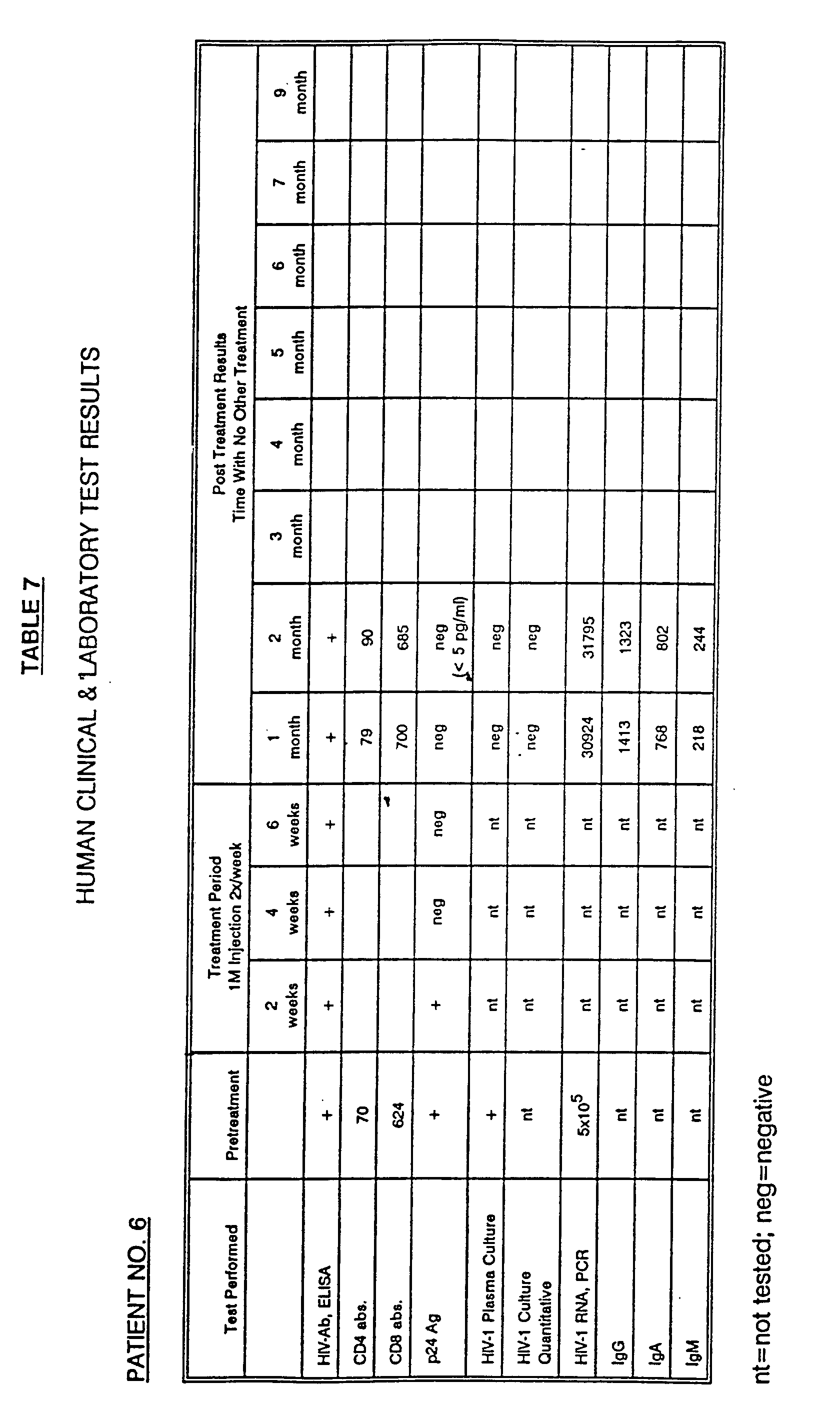

### Conclusion

This Example shows that HIV viral activity was stopped as seen by P24 core antigen converting to less that 5 *µ*g/ml, the limit of test method. Dramatic increase of CD4 count in the HIV patient was also shown. This increase is more dramatic over time than those observed for antiviral products commercially available in the United States, such as AZT and others. This Example also shows dramatic decreases in viral load of the patients as determined by quantitative RNA polymerase chain reaction. The patients' plasma cultures converted to negative. In ***in vitro*** systems, the patients' blood plasma was non-infective. The patients' T-cells were unable to infect non-infected (normal) patients' T-cells ***in vitro.*** The foregoing may indicate lack of virus in the plasma. It may also indicate the patient might have been immunized, and thus unable to infect other non-infected patients.

The peripheral blood mononucleocytes (PBMC) tested negative for HIV. The treatment transformed full blown AIDS patients to patients having undetectable HIV both in their plasma and peripheral blood cells. It appeared that both humoral and cellular immunity of the patient were utilized to destroy and neutralize the virus.

## Claims

1. A composition of TF protein comprising a lysine-rich histone fraction obtainable from a mammalian thymus cell nucleus, said composition when contacted with serum of a human, uninfected with HIV, is capable of precipitating β-, α₁- and α₂- macroglobulins of a healthy mammal.

2. The composition of claim 1, wherein the TF protein is unmethylated and comprises a protein of about 35 kilodalton as determined by 11 % non-reducing sodium dodecyl sulfate-polyacrylamide gel eiectrophoresis.

3. The composition of claim 1, wherein the TF protein is methylated and comprises a protein of about 23 kilodalton as determined by 11% non-reducing sodium dodecyl sulfate-polyacrylamide gel electrophoresis.

4. The composition of claim 1, wherein the TF protein has an isoelectric point of about 5.6.

5. The composition of claim 4, wherein the composition has a pH of between about 7.64 and 8.6.

6. A method for preparing a composition of TF protein comprising the steps of:
(a) purifying a lysine-rich histone fraction from mammalian thymus cell nuclei; and
(b) contacting the lysine-rich histone fraction with a cation exchange chromatographic material for a time sufficient for the TF protein to bind to the material;
(c) eluting the TF protein from the cation chromatographic material by contacting the chromatographic material with an effective aqueous salt solution;

7. The method of claim 6, further comprising the step of collecting the eluate that is capable of precipitating β-, α₁- and α₂- macroglobulins of a healthy mammal.

8. The method of claim 7, wherein the cation exchange chromatographic material is a carboxymethyl column chromatography and the carboxymethyl column is washed and the TF protein is eluted by applying a linear gradient of sodium chloride in 0.05 M sodium acetate at pH 6.8.

9. The method of claim 7, wherein the lysine-rich histone fraction is obtained by deaggregating the cell nuclei by enzymatic treatment with pepsin and isolating from the resulting deaggregating mixture the lysine-rich histone fraction.

10. A method for detecting an infection, in a human subject, comprising the steps of:
(a) collecting a biological sample from the subject, and
(b) detecting the pattern of fragmentation of a protein, aTF, which is capable of binding a protein TF present in a lysine-rich histone fraction obtainable from a thymus cell nucleus, wherein said infection results in the fragmentation of aTF.

11. The method of claim 10, wherein TF is capable of precipitating β-, α₁- and α₂- macroglobulins of a healthy mammal.

12. The method of claim 11, wherein TF is of about 35 kilodalton when unmethylated, and of about 28 kilodalton when methylated, as determined by 11% non-reducing sodium dodecyl sulfate-polyacrylamide gel electrophoresis.

13. The method of claim 12, wherein the pattern of fragmentation of aTF is detected by its precipitation pattern with TF.

14. The method of claim 13, wherein the method comprises two-dimensional gel electrophoresis, and the precipitation pattern of aTF and TF is further detected by its location in relation to β-, α₁- and *α*₂-macroglobulins, on the gel electrophoresis.

15. The method of claim 14, wherein said biological sample is selected from the group consisting of: serum, blood and plasma.

16. A method for detecting HIV infection in a human subject, comprising the steps of:
a) collecting a biological sample from the subject, said biological sample being selected from the group consisting of: serum, plasma and blood;
b) mixing the biological sample with a protein, TF, purified from a lysine-rich histone fraction of non-human mammalian thymus nuclei,
c) correlating precipitation patterns of TF and β-, α₁- and α₂- macroglobulins from the biological sample, with HIV-infection.

17. The method of claim 16, wherein the method comprises applying the biological sample and TF to gel electrophoresis, and correlating the precipitation pattern of TF and the biological sample, in relation to the location of β-, α₁- and α₂- macroglobulins on the gel, with HIV infection.

18. The method of claim 17, wherein the TF is further methylated and has a molecular weight of about 28 kilodalton as determined by 11% non-reducing sodium dodecyl sulfate-polyacrylamide get electrophoresis, and an isoelectric point of about 5.6.

19. The method of claim 18, wherein the gel electrophoresis is a two-dimensional gel electrophoresis.

20. The method of claim 19, wherein the absence of a continuous precipitation spur next to and from the positions of β-, α- to α₂- macroglobulins on the gel indicates HIV infection in the human subject.

21. The method of claim 20, wherein if the continuous precipitation spur is absent, but broken precipitation spurs are observed, then the human subject has a more advanced HIV-infection or HIV-related disease if the precipitation spurs are fainter or of lesser molecular weight.

22. A device for ***in vitro*** detection of HIV infection in a human subject, said device comprising a solid support containing a region for dispensing a biological fluid sample from the human subject, and a region for dispensing a protein TF; said TF being capable of extraction from a lysine-rich portion of non-human mammalian thymus cell nuclei, and capable of precipitating β-, α₁ - and α₂ - macroglobulins from a serum sample of a human uninfected with HIV; said solid support allows the biological sample to contact and precipitate with TF and the detection of such precipitation.

23. The device of claim 22, wherein said solid support is a two-dimensional electrophoretic gel and said contact and precipitation are achieved by means of two-dimensional gel electrophoresis, and said biological sample is selected from the group consisting of: blood, serum and plasma.

24. A kit useful for ***in vitro*** detection of HIV infection in a human subject, said kit comprising:
a) a container containing a protein TF, capable of extraction from a lysine-rich portion of non-human mammalian thymus cell nuclei, and capable of precipitating β-, α₁- and α₂- macroglobulins from a serum sample of a human uninfected with HIV; and
b) a device with an electrophoretic gel suitable for conducting a two-dimensional electrophoresis.

25. A method for purifying a protein TF, comprising the steps of:
a) extracting cell nuclei from mammalian thymus;
b) obtaining a lysine-rich portion from the cell nuclei; and
c) purifying TF from the lysine-rich portion based on TF's ability to precipitate β-, α₁- and α₂-macroglobulins.

26. A first protein possessing the properties of a second protein, said second protein being extractable from a lysine-rich histone fraction of a mammalian thymus cell nucleus, said properties possessed by the first and second protein being their
a) ability to precipitate a single continuous precipitation spur, on a two-dimensional electrophoretic gel, adjacent to β-, α₁- and α₂- macroglobulins from serum of a healthy human; and
b) inability to precipitate a single continuous precipitation spur, on a two-dimensional electrophoretic gel, adjacent to β-, α₁- and α₂- macroglobulins from serum of an AIDS human patient.

27. The first protein of Claim 26, wherein the second protein is extractable from the mammalian thymus cell nucleus by means of pepsin degradation.

28. The use of a composition comprising the first and/or second protein of Claim 26 for preparing a medicament for treating a human suffering from HIV infection.

29. The use of Claim 28, wherein the HIV infection is selected from the group consisting of AIDS and ARC; the second protein is extractable from the mammalian thymus cell nucleus by pepsin degradation, and the mammalian thymus cell nucleus is non-human.

30. The use of a composition comprising the first and/or second protein of Claim 26 for preparing a vaccine for vaccinating a human against HIV infection.

## Patentansprüche

1. Eine TF-Protein-Zusammensetzung umfassend einen lysinreichen Histonanteil, herstellbar aus einem Thymuszellkern eines Säugetiers, wobei die genannte Zusammensetzung die Fähigkeil hat, β-, α₁- und α₂- Makroglobuline eines gesunden Säugetiers zu präzipitieren, wenn sie in Kontakt mit einem menschlichen Serum, das nicht HIV infiziert ist, kommt.

2. Die Zusammensetzung nach Anspruch 1, wobei das TF-Protein nicht methyliert ist und ein Protein mit einem Gewicht von ca. 35 Kilodalton umfasst, bestimmt durch eine 11%ige nicht reduzierende Natriumdodecylsulfat-Polyacrylamidgel-Elektrophorese.

3. Die Zusammensetzung nach Anspruch 1, wobei das TF-Protein methyliert ist und ein Protein mit einem Gewicht von ca. 28 Kilodalton umfasst, bestimmt durch eine 11%ige nicht reduzierende Natriumdodecylsulfat-Polyacrylamidgel-Elektrophorese.

4. Die Zusammensetzung nach Anspruch 1, wobei das TF-Protein einen isoelektrischen Punkt von 5,6 hat.

5. Die Zusammensetzung nach Anspruch 4, wobei die Zusammensetzung einen pH-Wert zwischen 7,64 und 8,6 hat.

6. Ein Verfahren zur Herstellung einer TF-Protein-Zusammensetzung, welches die Schritte umfasst:
(a) Reinigen eines lysinreichen Histonanteils von Thymuszellkernen eines Säugetiers; und
(b) Kontaktieren des lysinreichen Histonanteils mit einem Kationenaustauschenden, chromatographischen Material, über eine Zeitspanne, die für das TF-Protein ausreicht, um das Material zu binden;
(c) Eluieren des TF-Proteins aus dem Kationen-chromatographischen-Material durch Kontaktieren des chromatographischen Materials mit einer wirksamen wässrigen Salzlösung.

7. Das Verfahren nach Anspruch 6, das weiters den Schritt des Sammelns des Eluats mit der Fähigkeit β-, α₁- und α₂- Makroglobuline eines gesunden Säugetiers zu präzipitieren, umfasst.

8. Das Verfahren nach Anspruch 7, wobei das Kationen-austauschende, chromatographische Material eine Carboxymethyl-Säulen-Chromatographie darstellt und die Carboxymethyl-Säule gewaschen und das TF-Protein durch Anwendung eines linearen Natriumchloridgradienten in 0,05 M Natriumacetat bei einem pH-Wert von 6,8 eluiert wird.

9. Das Verfahren nach Anspruch 7, wobei der lysinreiche Histonanteil durch Deaggregation der Zellkerne mittels enzymatischer Behandlung mit Pepsin erhalten wird und der lysinreiche Histonanteil von der erhaltenen Deaggregationsmischung abgesondert wird.

10. Ein Verfahren, bei einern Menschen eine Infektion festzustellen, welche die Schritte umfasst:
(a) Entnehmen einer biologischen Probe des Menschen, und
(b) Erfassen des Fragmentationsmusters eines Proteins, aTF, mit der Fähigkeit ein TF-Protein zu binden, das in einem lysinreichen Histonanteil, herstellbar aus einem Thymuszellkern, vorhanden ist, wobei die genannte Infektion die Fragmentation des aTF zur Folge hat.

11. Das Verfahren nach Anspruch 10, wobei das TF die Fähigkeit hat, β-, α₁- und α₂- Makroglobuline eines gesunden Säugetiers zu präzipitieren.

12. Das Verfahren nach Anspruch 11, wobei das TF unmethyliert eine Gewicht von ca. 35 Kilodalton umfasst, und methyliert ca. 28 Kilodalton umfasst, bestimmt durch eine 11%ige nicht reduzierende Natriumdodecylsulfat-Polyacrylamidgel-Elektrophorese.

13. Das Verfahren nach Anspruch 12, wobei das Fragmentationsmuster eines aTF mit Hilfe seines Präzipitationsmuster mit TF ermittelt wird.

14. Das Verfahren nach Anspruch 13, wobei das Verfahren eine zweidimensionale Gel-Elektrophorese umfasst, und die Präzipitationsmuster von aTF und TF weiters durch ihre Positionierung in Bezug auf die β-, α₁- und α₂- Makroglobuline auf der Gel-Elektrophorese ermittelt werden.

15. Das Verfahren nach Anspruch 14, wobei die genannte biologische Probe aus einer Gruppe ausgewählt ist, bestehend aus: Serum, Blut und Plasma.

16. Ein Verfahren für das Erkennen einer HIV-Infektion bei einer Person, die folgende Schritte umfasst:
a) Entnehmen einer biologischen Probe des Menschen, wobei die genannte biologische Probe aus einer Gruppe ausgewählt ist, bestehend aus: Serum, Plasma und Blut;
b) Mischen der biologischen Probe mit einem Protein, TF, das durch Reinigen eines lysinreichen Histonanteils von nicht-menschlichen Thymuszellkernen, die von einem Säugetier stammen, erhalten wird.
c) Korrelieren der- Präzipitationsmuster von TF und β-, α₁- und α₂-Makroglobulinen der biologischen Probe mit einer HIV-Infektion.

17. Das Verfahren nach Anspruch 16, wobei das Verfahren das Unterziehen der biologischen Probe und des TF einer Gelelektrophorese und das Korrelieren der Präzipitationsmuster von TF und biologischer Probe in Bezug auf ihre Positionierung der β-, α₁- und α₂- Makroglobuline auf dem Gel mit einer HIV-Infektion, umfasst.

18. Das Verfahren nach Anspruch 17, wobei das TF weiters methyliert wird und ein Molekulargewicht von ca. 28 Kilodalton aufweist, bestimmt durch eine 11%ige nicht reduzierende Natriumdodecylsulfat-Polyacrylamidgel-Elektrophorese, und über einen isoelektrischen Punkt von ca. 5,6 verfügt.

19. Das Verfahren nach Anspruch 18, wobei die Gelelektrophorese eine zweidimensionale Gelelektrophorese darstellt.

20. Das Verfahren nach Anspruch 19, wobei das Fehlen einer kontinuierlichen Präzipitationsspur neben und ausgehend von den Positiorien der β-, α- zu den α₂-Makroglobulinen auf dem Gel eine HIV-Infektion des Menschen anzeigt.

21. Ein Verfahren nach Anspruch 20, wobei der Mensch eine HIV-Infektion im fortgeschrittenem Stadium hat, wenn eine kontinuierliche Präzipitationsspur fehlt aber unterbrochene Präzipitationsspuren beobachtet werden, oder eine HIV-verwandte Krankheit, wenn die Präzipitationsspuren schwächer sind oder ein geringeres Molekulargewicht haben.

22. Eine Einrichtung für die *In-vitro*-Erkennung einer HIV-Infektion bei einem Menschen, wobei die genannte Einrichtung einen festen Träger umfasst, der einen Bereich für die Ausgabe der biologische Flüssigkeitsprobe des Menschen und einen Bereich für die Ausgabe des TF-Proteins enthält; wobei das genannte TF zur Extraktion aus einem lysinreichen Anteil eines nicht-menschlichen Thymuszellkerns eines Säugetiers fähig ist, und über die Fähigkeit verfügt, β-, α₁- und α₂-Makroglobuline aus einer menschlichen Serumsprobe, die nicht HIV infiziert ist, zu präzipitieren; wobei der genannte feste Träger ermöglicht, die biologische Probe mit dem TF in Kontakt zu bringen und zu präzipitieren und die Ermittlung einer solchen Präzipitation.

23. Die Einrichtung nach Anspruch 22, wobei der feste Träger ein zweidimensionales elektrophoretisches Gel darstellt und das genannte Kontaktieren und die Präzipitation mittels einer zweidimensionalen Gelelektrophorese erreicht werden, und die genannte biologische Probe ausgewählt ist aus einer Gruppe bestehend aus: Blut, Serum und Plasma.

24. Ein Set zweckmäßig für die *In-vitro*-Erkennung einer HIV-Infektion bei einem Menschen, wobei genanntes Set umfasst:
a) einen Behälter, der ein TF-Protein enthält mit der Fähigkeit zur Extraktion aus einem lysinreichen Anteil von nicht-menschlichen Thymuszellkernen eines Säugetiers, und mit der Fähigkeit, β-, α₁- und α₂- Makroglobuline aus einer menschlichen Serumsprobe, die nicht HIV infiziert ist, zu präzipitieren; und
b) eine Einrichtung mit einem elektrophoretischen Gel, das für die Durchführung einer zweidimensionalen Elektrophorese geeignet ist.

25. Ein Verfahren für das Reinigen des TF-Proteins, das folgende Schritte umfasst:
a) Extrahieren der Zellkerne aus dem Thymus eines Säugestiers;
b) Erhalten eines lysinreichen Anteils aus dem Zellkern; und
c) Reinigen des TF von dem lysinreichen Anteil basierend auf der TF-Fähigkeit β-, α₁- und α₂- Makroglobuline zu präzipitieren.

26. Ein erstes Protein, das die Eigenschaften eines zweiten Proteins besitzt, wobei das zweite Protein extrahierbar aus einem lysinreichen Histonanteil von einem Thymuszellkern eines Säugetiers ist, und die genannten Eigenschaften, die das erste und zweite Protein besitzen, ihre
a) Fähigkeit, eine einzige kontinuierliche Präzipitationspur zu präzipitieren, auf einem zweidimensionalen elektrophoretischen Gel, angrenzend an β-, α₁- und α₂- Makroglobuline aus dem Serum eines gesunden Menschen; und
b) Unfähigkeit, eine einzige kontinuierliche Präzipitationsspur zu präzipitieren, auf einem zweidimensionalen elektrophoretischen Gel, angrenzend an β-, α₁- und α₂- Makroglobuline aus dem Serum eines menschlichen AIDSPatienten.
sind.

27. Das erste Protein nach Anspruch 26, wobei das zweite Protein extrahierbar aus einem Thymuszellkern eines Säugetiers durch Pepsindegradation ist.

28. Die Anwendung einer Zusammensetzung, welche das erste und/oder zweite Protein nach Anspruch 26 für die Herstellung eines Medikaments für die Behandlung eines HIV-infizierten Menschen umfasst.

29. Die Anwendung nach Anspruch 28, wobei die HIV-Infektion ausgewählt ist aus der Gruppe bestehend aus AIDS und ARC; das zweite Protein ist extrahierbar aus dem Thymuszellkern eines Säugetiers durch Pepsindegradation, und der Thymuszellkern des Säugetiers stammt nicht von einem Menschen.

30. Die Anwendung einer Zusammmensetzung, welche das erste und/oder zweite Protein nach Anspruch 26 für die Herstellung eines Impfstoffes für die Impfung eines Menschen gegen eine HIV-Infektion umfasst.

## Revendications

1. Composition de facteur thymique (TF) comprenant une fraction histone riche en lysine que l'on peut obtenir à partir de noyaux de cellules thymiques de mammifères, ladite composition, lorsqu'on la met en contact avec du sérum humain, non infecté par le VIH, étant capable de faire précipiter les macroglobulines β, α₁ et α₂ d'un mammifère en bonne santé.

2. Composition selon la revendication 1, dans laquelle le facteur thymique est non méthylé et comprend une protéine d'environ 35 kilodaltons, masse déterminée par électrophorèse sur gel de polyacrylamide à 11 % en présence de dodécylsulfate de sodium dans des conditions non réductrices.

3. Composition selon la revendication 1, dans laquelle le facteur thymique est méthylé et comprend une protéine d'environ 28 kilodaltons, masse déterminée par électrophorèse sur gel de polyacrylamide à 11 % en présence de dodécylsulfate de sodium dans des conditions non réductrices.

4. Composition selon la revendication 1, dans laquelle le facteur thymique a un point isoélectrique d'environ 5,6.

5. Composition selon la revendication 4, dans laquelle la composition a un pH compris entre environ 7,64 et 8,6.

6. Procédé de préparation d'une composition de facteur thymique comprenant les étapes consistant
(a) à purifier une fraction histone riche en lysine à partir de noyaux de cellules thymiques de mammifères, et
(b) à mettre en contact la fraction histone riche en lysine avec un matériau chromatographique échangeur de cations pendant une durée suffisante pour que le facteur thymique se fixe sur le matériau,
(c) à éluer le facteur thymique du matériau chromatographique échangeur de cations en mettant en contact le matériau chromatographique avec une solution saline aqueuse efficace.

7. Procédé selon la revendication 6, comprenant en outre l'étape consistant à recueillir les fractions éluées capables de faire précipiter les macroglobulines β, α₁ et α₂ d'un mammifère en bonne santé.

8. Procédé selon la revendication 7, dans lequel le matériau chromatographique échangeur de cations est une colonne chromatographique carboxyméthylée et la colonne carboxyméthylée est lavée et le facteur thymique est élué par application d'un gradient linéaire de chlorue de sodium dans une solution d'acétate de sodium à 0,05 M et à pH 6,8.

9. Procédé selon la revendication 7, dans lequel la fraction histone riche en lysine est obtenue par désagrégation des noyaux cellulaires par un traitement enzymatique avec la pepsine et isolement de la fraction histone riche en lysine à partir du mélange désagrégé obtenu.

10. Procédé de détection d'une infection chez un sujet humain, comprenant les étapes consistant
(a) à prélever un échantillon biologique d'un sujet, et
(b) à observer le profile de fragmentation d'une protéine, appelée aTF, capable de se lier au facteur thymique présent dans une fraction histone riche en lysine que l'on peut obtenir à partir de noyaux de cellules thymiques, ladite infection se traduisant par la fragmentation de la protéine aTF.

11. Procédé selon la revendication 10, dans lequel le facteur thymique est capable de faire précipiter les macroglobulines β, α₁ et α₂ d'un mammifère en bonne santé.

12. Procédé selon la revendication 11, dans lequel le facteur thymique a une masse d'environ 35 kilodaltons lorsqu'il est non-méthylé, et d'environ 28 kilodaltons lorsqu'il est méthylé, masses déterminées par électrophorèse sur gel de polyacrylamide à 11 % en présence de dodécylsulfate de sodium dans des conditions non réductrices.

13. Procédé selon la revendication 12, dans lequel le profile de fragmentation de la protéine aTF est détecté grâce à son profile de précipitation avec le facteur thymique.

14. Procédé selon la revendication 13, dans lequel le procédé comprend une étape d'électrophorèse bidimensionnelle sur gel, et le profile de précipitation de la protéine aTF et du facteur thymique est en outre détecté grâce à sa position par rapport aux macroglobulines β, α₁ et α₂ sur le gel d'électrophorèse.

15. Procédé selon la revendication 14, dans lequel ledit échantillon biologique est choisi dans le groupe formé par le sérum, le sang et le plasma.

16. Procédé de détection d'une infection par le VIH chez un sujet humain, comprenant les étapes consistant :
(a) à prélever un échantillon biologique d'un sujet, ledit échantillon biologique étant choisi dans le groupe formé par le sérum, le plasma et le sang ;
(b) à mélanger l'échantillon biologique avec une protéine, le facteur thymique, purifiée à partir d'une fraction histone riche en lysine de noyaux de cellules thymiques de mammifères non humains ;
(c) à comparer les profiles de précipitation du facteur thymique et des macroglobulines β, α₁ et α₂ de l'échantillon biologique avec celui d'un échantillon infecté par le VIH.

17. Procédé selon la revendication 16, comprenant une étape d'électrophorèse sur gel de l'échantillon biologique et du facteur thymique, et la comparaison du profile de précipitation du facteur thymique et de l'échantillon biologique, en ce qui concerne la position des macroglobulines β, α₁ et α₂ sur le gel, avec celui d'un échantillon infecté par le VIH.

18. Procédé selon la revendication 17, dans lequel le facteur thymique est en outre méthylé et a une masse moléculaire d'environ 28 kilodaltons, masse déterminée par électrophorèse sur gel de polyacrylamide à 11 % en présence de dodécylsulfate de sodium dans des conditions non réductrices et a un point isoélectrique d'environ 5,6.

19. Procédé selon la revendication 18, dans lequel l'électrophorèse sur gel est une électrophorèse bidimensionnelle sur gel.

20. Procédé selon la revendication 19, dans lequel l'absence d'une trace de précipitation continue au voisinage et à partir des positions des macroglobulines β, α₁ et α₂ sur le gel indique une infection du sujet humain par le VIH.

21. Procédé selon la revendication 20, dans lequel, lorsque la trace de précipitation continue est absente, mais lorsqu'on observe des traces de précipitation interrompues, alors le sujet humain a une infection à VIH ou une maladie associée au VIH plus avancée lorsque les traces de précipitation sont plus faibles ou situées à des masses moléculaires moins élevées.

22. Dispositif de détection *in vitro* d'une infection par le VIH d'un sujet humain, ledit dispositif comprenant un support solide avec une zone pour dispenser un échantillon biologique fluide du sujet humain et une zone pour dispenser du facteur thymique, ledit facteur thymique pouvant être extrait de la fraction histone riche en lysine de noyaux de cellules thymiques de mammifères non-humains, et étant capable de faire précipiter les macroglobulines β, α₁ et α₂ d'un échantillon de sérum d'un être humain non infecté par le VIH, ledit support solide permettant la mise en contact de l'échantillon biologique avec le facteur thymique et la précipitation de ceux-ci, et la détection d'une telle précipitation.

23. Dispositif selon la revendication 22, dans lequel ledit support solide est un gel pour électrophorèse bidimensionnelle et ladite mise en contact et ladite précipitation sont obtenues au moyen d'une électrophorèse bidimensionnelle sur gel, et ledit échantillon biologique est choisi dans le groupe formé par le sang, le sérum et le plasma.

24. Kit utilisable pour la détection *in vitro* d'une infection à VIH chez un sujet humain, ledit kit comprenant :
(a) un récipient contenant du facteur thymique, que l'on peut extraire de la fraction histone riche en lysine de noyaux de cellules thymiques de mammifères non-humains, et qui est capable de faire précipiter les macroglobulines β, α₁ et α₂ d'un échantillon de sérum d'un être humain non infecté par le VIH, et
(b) un dispositif avec un gel d'électrephorèse permettant de réaliser une électrophorèse bidimensionnelle.

25. Procédé de purification du facteur thymique, comprenant les étapes consistant
(a) à extraire des noyaux de cellules de thymus de mammifères,
(b) à obtenir une fraction riche en lysine des noyaux de cellules, et
(c) à purifier le facteur thymique de la fraction riche en lysine grâce à la capacité du facteur thymique de faire précipiter les macroglobulines β, α₁ et α₂.

26. Une première protéine possédant des propriétés d'une seconde protéine, ladite seconde protéine pouvant être extraite d'une fraction histone riche en lysine de noyaux de cellules thymiques de mammifères, lesdites propriétés que possèdent la première et la seconde protéines étant
(a) leur capacité à précipiter sous forme d'une trace de précipitation continue unique, sur un gel d'électrophorèse bidimensionnelle, au voisinage des macroglobulines β, α₁ et α₂ à partir de sérums d'êtres humains en bonne santé, et
(b) leur incapacité à précipiter sous forme d'une trace de précipitation continue unique, sur un gel d'électrophorèse bidimensionnelle, au voisinage des macroglobulines β, α₁ et α₂ à partir de sérums de patients humains malades du SIDA.

27. Une première protéine selon la revendication 26, la seconde protéine pouvant être extraite de noyaux de cellules thymiques de mammifères après dégradation par la pepsine.

28. Utilisation d'une composition comprenant la première et/ou la seconde protéine selon la revendication 26 pour la préparation d'un médicament destiné au traitement de patients souffrant d'une infection par le VIH.

29. Utilisation selon la revendication 28, dans laquelle l'infection par le VIH est choisie dans le groupe formé par le SIDA et le complexe lié au SIDA, la seconde protéine pouvant être extraite de noyaux de cellules thymiques de mammifères après dégradation par la pepsine et les noyaux de cellules thymiques de mammifères étant des noyaux de cellules thymiques non-humains.

30. Utilisation d'une composition comprenant la première et/ou la seconde protéine selon la revendication 26 pour préparer un vaccin pour vacciner des êtres humains contre l'infection par le VIH.
